# EUROPEAN PATENT APPLICATION

(11) **EP 2 837 694 A1**
(43) Date of publication of application: **18.02.2015**
(21) Application number: 13180632.5
(22) Date of filing: 16.08.2013
(51) Int. Cl.: C12Q 1/68, C12N 15/113, G01N 33/574

(54) **Method of diagnosing cancer based on MED15 and/or MED12**

(71) Applicant: Rheinische Friedrich-Wilhelms-Universität Bonn, 53113 Bonn (DE)
(72) Inventor: Perner, Sven, 53127 Bonn (DE); Shaikhibrahim, Zaki, 53127 Bonn (DE); Menon, Roopika, 50969 Köln (DE); Braun, Martin, 53115 Bonn (DE)
(74) Representative: Viering, Jentschura & Partner Patent- und Rechtsanwälte

(57) **Abstract**

The present invention relates to methods of diagnosing cancer, determining the prognosis of a patient diagnosed with cancer and identifying a compound for treatment of cancer, using MED15 and/or MED12 as markers. In particular, the cancer is prostate cancer, castration-resistant prostate cancer (CRPC), distant metastatic CRPC or local recurrent CRPC. Furthermore, the invention relates to compounds for the treatment of cancer and use of MED15 and/or MED12 for diagnosis of cancer or prognosis of a patient suffering from cancer.

SEQUENCE LISTING

<110>  Universität Bielefeld

<120>  Method of diagnosis cancer

<130>  P53738

<160>  246

<170>  PatentIn version 3.5

<210>  1
<211>  21
<212>  DNA
<213>  Artificial Sequence

<220>
<223>  Sequencing Primer

<400>  1
cttcccgttc ccccagtcag c                                                21

<210>  2
<211>  21
<212>  DNA
<213>  Artificial Sequence

<220>
<223>  Sequencing Primer

<400>  2
gcccctccag agccccgatt t                                                21

<210>  3
<211>  23
<212>  DNA
<213>  Artificial Sequence

<220>
<223>  Sequencing Primer

<400>  3
   caccccttcc cccttcccct aag                                             23

<210>  4
<211>  26
<212>  DNA
<213>  Artificial Sequence

<220>
<223>  Sequencing Primer

<400>  4
tttgctcctt cttagattct tccagc                                           26

<210>  5
<211>  24
<212>  DNA

<213>  Artificial Sequence

<220>
<223>  Sequencing Primer

<400>  5
ccagcaaaag gcaggaccac ctgt                                              24


<210>  6
<211>  24
<212>  DNA
<213>  Artificial Sequence

<220>
<223>  Sequencing Primer

<400>  6
gcactctggg atcgtgcagt cacc                                              24


<210>  7
<211>  23
<212>  DNA
<213>  Artificial Sequence

<220>
<223>  Sequencing Primer

<400>  7
gcaggaagcc ccaagtgaac cag                                               23


<210>  8
<211>  24
<212>  DNA
<213>  Artificial Sequence

<220>
<223>  Sequencing Primer

<400>  8
tccttggcct gaaggacagg aaac                                              24


<210>  9
<211>  32
<212>  DNA
<213>  Artificial Sequence

<220>
<223>  Sequencing Primer

<400>  9
tcctattccc atattaagct acatgggtgt ca                                     32


<210>  10
<211>  28
<212>  DNA
<213>  Artificial Sequence

<220>

<223>  Sequencing Primer

<400>  10
tcagagattg ctgcatagta ggcacagg                                          28


<210>  11
<211>  31
<212>  DNA
<213>  Artificial Sequence

<220>
<223>  Sequencing Primer

<400>  11
ccattttcag taagaaggaa gaggtgtttg g                                      31


<210>  12
<211>  26
<212>  DNA
<213>  Artificial Sequence

<220>
<223>  Sequencing Primer

<400>  12
ctccttgaag ccatcagcag tacacc                                            26


<210>  13
<211>  41
<212>  DNA
<213>  Artificial Sequence

<220>
<223>  Sequencing Primer

<400>  13
gtagaaggat attctaagca gaaggatagt atcaaatagc c                           41


<210>  14
<211>  24
<212>  DNA
<213>  Artificial Sequence

<220>
<223>  Sequencing Primer

<400>  14
gccacctcta catcatgggg caag                                              24


<210>  15
<211>  26
<212>  DNA
<213>  Artificial Sequence

<220>
<223>  Sequencing Primer

<400>  15

tgggagcagt tacagaagat ggctga 26

<210> 16
<211> 23
<212> DNA
<213> Artificial Sequence

<220>
<223> Sequencing Primer

<400> 16
aagctcaacc caatgcccca cac 23

<210> 17
<211> 34
<212> DNA
<213> Artificial Sequence

<220>
<223> Sequencing Primer

<400> 17
tttatctgct tcatctctaa tagtcccctc ttcc 34

<210> 18
<211> 24
<212> DNA
<213> Artificial Sequence

<220>
<223> Sequencing Primer

<400> 18
cccctgcccc accagtacca aaat 24

<210> 19
<211> 25
<212> DNA
<213> Artificial Sequence

<220>
<223> Sequencing Primer

<400> 19
ccctggagag aactaggggc tctga 25

<210> 20
<211> 24
<212> DNA
<213> Artificial Sequence

<220>
<223> Sequencing Primer

<400> 20
ggtgggtagc gtgcttgttg actg 24

<210>  21
<211>  23
<212>  DNA
<213>  Artificial Sequence

<220>
<223>  Sequencing Primer

<400>  21
ccgccggctt gcctacttct gta                                              23


<210>  22
<211>  21
<212>  DNA
<213>  Artificial Sequence

<220>
<223>  Sequencing Primer

<400>  22
aaaaaccagg ggccggtgct g                                                21


<210>  23
<211>  24
<212>  DNA
<213>  Artificial Sequence

<220>
<223>  Sequencing Primer

<400>  23
ctcagccccc aactagcagc acac                                             24


<210>  24
<211>  25
<212>  DNA
<213>  Artificial Sequence

<220>
<223>  Sequencing Primer

<400>  24
tgggcacctg attcttctca ggcta                                            25


<210>  25
<211>  26
<212>  DNA
<213>  Artificial Sequence

<220>
<223>  Sequencing Primer

<400>  25
ggggtaacca tggtgaatga gttgga                                           26


<210>  26
<211>  24
<212>  DNA

<213>  Artificial Sequence

<220>
<223>  Sequencing Primer

<400>  26
gttaccctct ggcatgggca ggtt                                              24


<210>  27
<211>  25
<212>  DNA
<213>  Artificial Sequence

<220>
<223>  Sequencing Primer

<400>  27
cctcctgtgc tgtcctagtg ccttg                                             25


<210>  28
<211>  27
<212>  DNA
<213>  Artificial Sequence

<220>
<223>  Sequencing Primer

<400>  28
tcatagccca atctgagagt accaggc                                           27


<210>  29
<211>  27
<212>  DNA
<213>  Artificial Sequence

<220>
<223>  Sequencing Primer

<400>  29
ctagcctggg gctctggcct tttatat                                           27


<210>  30
<211>  24
<212>  DNA
<213>  Artificial Sequence

<220>
<223>  Sequencing Primer

<400>  30
ccagcgaact tcaactgcct gtcc                                              24


<210>  31
<211>  24
<212>  DNA
<213>  Artificial Sequence

<220>

<223>  Sequencing Primer

<400>  31
gggagatcga gcagcagatc aagg                                              24


<210>  32
<211>  31
<212>  DNA
<213>  Artificial Sequence

<220>
<223>  Sequencing Primer

<400>  32
gtgcattctg gctcttagat ctacatgtcc t                                      31


<210>  33
<211>  31
<212>  DNA
<213>  Artificial Sequence

<220>
<223>   Sequencing Primer

<400>  33
aggacatgta gatctaagag ccagaatgca c                                      31


<210>  34
<211>  26
<212>  DNA
<213>  Artificial Sequence

<220>
<223>  Sequencing Primer

<400>  34
ggtcccaatc caaagattcg gttaca                                            26


<210>  35
<211>  27
<212>  DNA
<213>  Artificial Sequence

<220>
<223>  Sequencing Primer

<400>  35
ggacagccat agttttgaac gctctga                                           27


<210>  36
<211>  24
<212>  DNA
<213>  Artificial Sequence

<220>
<223>  Sequencing Primer

<400>  36

tgctctgctt tcaatgcctc acca                                              24

<210>  37
<211>  25
<212>  DNA
<213>  Artificial Sequence

<220>
<223>  Sequencing Primer

<400>  37
tcatttcttt gtccccaccc ctacc                                             25

<210>  38
<211>  24
<212>  DNA
<213>  Artificial Sequence

<220>
<223>  Sequencing Primer

<400>  38
tcagcctcaa tctccgcctg tctc                                              24

<210>  39
<211>  27
<212>  DNA
<213>  Artificial Sequence

<220>
<223>  Sequencing Primer

<400>  39
ggtgtcattg ctatgtgaat gggctgt                                           27

<210>  40
<211>  24
<212>  DNA
<213>  Artificial Sequence

<220>
<223>  Sequencing Primer

<400>  40
ggtccctcca ccacctccat ctct                                              24

<210>  41
<211>  31
<212>  DNA
<213>  Artificial Sequence

<220>
<223>  Sequencing Primer

<400>  41
cattgaggag tgaagaagca ggtaaaagtc a                                      31

<210>  42
<211>  24
<212>  DNA
<213>  Artificial Sequence

<220>
<223>  Sequencing Primer

<400>  42
gcatgggagc ctgtgtatcc agaa                                              24


<210>  43
<211>  23
<212>  DNA
<213>  Artificial Sequence

<220>
<223>  Sequencing Primer

<400>  43
atcgcctctg gctccctttc tgc                                               23


<210>  44
<211>  35
<212>  DNA
<213>  Artificial Sequence

<220>
<223>  Sequencing Primer

<400>  44
ctgcatagag cccaagagag aaatatagac cagag                                  35


<210>  45
<211>  28
<212>  DNA
<213>  Artificial Sequence

<220>
<223>  Sequencing Primer

<400>  45
ccttaggtgt gtccctctct cttttggc                                          28


<210>  46
<211>  24
<212>  DNA
<213>  Artificial Sequence

<220>
<223>  Sequencing Primer

<400>  46
ccctcgggag atgagagtgc aagt                                              24


<210>  47
<211>  24
<212>  DNA

<213>  Artificial Sequence

<220>
<223>  Sequencing Primer

<400>  47
cggaccctcg aagtgagagt gagc                                              24


<210>  48
<211>  24
<212>  DNA
<213>  Artificial Sequence

<220>
<223>  Sequencing Primer

<400>  48
ggtcatcggc aggatcatca aagg                                              24


<210>  49
<211>  22
<212>  DNA
<213>  Artificial Sequence

<220>
<223>  Sequencing Primer

<400>  49
cttgcctttg gagcccctgg tc                                                22


<210>  50
<211>  29
<212>  DNA
<213>  Artificial Sequence

<220>
<223>  Sequencing Primer

<400>  50
ccatgaagat ggaaatgtca gaagaaacg                                         29


<210>  51
<211>  24
<212>  DNA
<213>  Artificial Sequence

<220>
<223>  Sequencing Primer

<400>  51
tgggagccac tccctagggc taaa                                              24


<210>  52
<211>  27
<212>  DNA
<213>  Artificial Sequence

<220>

<223>  Sequencing Primer

<400>  52
tgggaagatc caaggatcca gattctg                                          27


<210>  53
<211>  25
<212>  DNA
<213>  Artificial Sequence

<220>
<223>  Sequencing Primer

<400>  53
gccccctcat ccactttcct tcttc                                            25


<210>  54
<211>  21
<212>  DNA
<213>  Artificial Sequence

<220>
<223>  Sequencing Primer

<400>  54
actgcacgtg tcgtggctgg t                                                21


<210>  55
<211>  24
<212>  DNA
<213>  Artificial Sequence

<220>
<223>  Sequencing Primer

<400>  55
tcccctgaga agccagatgt cgag                                             24


<210>  56
<211>  25
<212>  DNA
<213>  Artificial Sequence

<220>
<223>  Sequencing Primer

<400>  56
cccctcagca aattcctttg acagc                                            25


<210>  57
<211>  26
<212>  DNA
<213>  Artificial Sequence

<220>
<223>  Sequencing Primer

<400>  57

catgctttca agaggaggga aggaga                                          26

<210>  58
<211>  22
<212>  DNA
<213>  Artificial Sequence

<220>
<223>  Sequencing Primer

<400>  58
tgctgtccct ttgcggttca ga                                              22

<210>  59
<211>  24
<212>  DNA
<213>  Artificial Sequence

<220>
<223>  Sequencing Primer

<400>  59
cgtactgttt ggggtgggaa agca                                            24

<210>  60
<211>  24
<212>  DNA
<213>  Artificial Sequence

<220>
<223>  Sequencing Primer

<400>  60
gggcattctt tgggggagat ctgt                                            24

<210>  61
<211>  30
<212>  DNA
<213>  Artificial Sequence

<220>
<223>  Sequencing Primer

<400>  61
cctgagagct ctagtccttt tgaaacttcc                                      30

<210>  62
<211>  31
<212>  DNA
<213>  Artificial Sequence

<220>
<223>  Sequencing Primer

<400>  62
ggaatatgta cagggaggtg ctaggttcag g                                    31

<210>  63
<211>  24
<212>  DNA
<213>  Artificial Sequence

<220>
<223>  Sequencing Primer

<400>  63
   cataccccac tccccacccc tagt                                          24

<210>  64
<211>  24
<212>  DNA
<213>  Artificial Sequence

<220>
<223>  Sequencing Primer

<400>  64
gagccgtgac ctggtgttgg tcat                                          24

<210>  65
<211>  21
<212>  DNA
<213>  Artificial Sequence

<220>
<223>  Sequencing Primer

<400>  65
gtggggagga tgggcagaag c                                             21

<210>  66
<211>  24
<212>  DNA
<213>  Artificial Sequence

<220>
<223>  Sequencing Primer

<400>  66
aacaggaggc cagaatgtgg gaaa                                          24

<210>  67
<211>  24
<212>  DNA
<213>  Artificial Sequence

<220>
<223>  Sequencing Primer

<400>  67
tccactccca tctcactccc actg                                          24

<210>  68
<211>  24
<212>  DNA

<213>  Artificial Sequence

<220>
<223>  Sequencing Primer

<400>  68
ggcaaagtcg atgaggccac tgat                                              24


<210>  69
<211>  25
<212>  DNA
<213>  Artificial Sequence

<220>
<223>  Sequencing Primer

<400>  69
ggcatgtcat accacttgcc tctgg                                             25


<210>  70
<211>  24
<212>  DNA
<213>  Artificial Sequence

<220>
<223>  Sequencing Primer

<400>  70
gctggagcct ctacgcctgt cctt                                              24


<210>  71
<211>  24
<212>  DNA
<213>  Artificial Sequence

<220>
<223>  Sequencing Primer

<400>  71
aggggcctct ttgcatttct cacc                                              24


<210>  72
<211>  24
<212>  DNA
<213>  Artificial Sequence

<220>
<223>  Sequencing Primer

<400>  72
tgccatctgg tcctggttga ggat                                              24


<210>  73
<211>  24
<212>  DNA
<213>  Artificial Sequence

<220>

<223>  Sequencing Primer

<400>  73
tcggatctgg tgggcagcta cact                                              24


<210>  74
<211>  24
<212>  DNA
<213>  Artificial Sequence

<220>
<223>  Sequencing Primer

<400>  74
ggcattcgcc agagctttgt ttca                                              24


<210>  75
<211>  24
<212>  DNA
<213>  Artificial Sequence

<220>
<223>  Sequencing Primer

<400>  75
gtttggagga gcccgcatac catc                                              24


<210>  76
<211>  29
<212>  DNA
<213>  Artificial Sequence

<220>
<223>  Sequencing Primer

<400>  76
gctccccaaa tttgttcttt aaatggcta                                         29


<210>  77
<211>  23
<212>  DNA
<213>  Artificial Sequence

<220>
<223>  Sequencing Primer

<400>  77
gcgtcgtgaa gcatgggatg aac                                               23


<210>  78
<211>  24
<212>  DNA
<213>  Artificial Sequence

<220>
<223>  Sequencing Primer

<400>  78

cctgagttgg gtggtcatgg gaag                                              24

<210>  79
<211>  34
<212>  DNA
<213>  Artificial Sequence

<220>
<223>  Sequencing Primer

<400>  79
caggccttct tcaacactac tatctccttt cctc                                    34

<210>  80
<211>  34
<212>  DNA
<213>  Artificial Sequence

<220>
<223>  Sequencing Primer

<400>  80
gttagcaggg ttctcactaa gactcttgcc tagc                                    34

<210>  81
<211>  24
<212>  DNA
<213>  Artificial Sequence

<220>
<223>  Sequencing Primer

<400>  81
tgggcacctg agttcatgat cgac                                              24

<210>  82
<211>  23
<212>  DNA
<213>  Artificial Sequence

<220>
<223>  Sequencing Primer

<400>  82
catggtgccc ttcctcactc agt                                               23

<210>  83
<211>  28
<212>  DNA
<213>  Artificial Sequence

<220>
<223>  Sequencing Primer

<400>  83
ggggcagaga tgagaagtta atgggtct                                          28

<210>  84
<211>  25
<212>  DNA
<213>  Artificial Sequence

<220>
<223>  Sequencing Primer

<400>  84
tgcagaggag atcgttgaaa ccaca                                              25


<210>  85
<211>  25
<212>  DNA
<213>  Artificial Sequence

<220>
<223>  Sequencing Primer

<400>  85
gctgaccggc tattgcaagt cactg                                              25


<210>  86
<211>  26
<212>  DNA
<213>  Artificial Sequence

<220>
<223>  Sequencing Primer

<400>  86
tctgtccctg gtcagaggat cacaac                                             26


<210>  87
<211>  24
<212>  DNA
<213>  Artificial Sequence

<220>
<223>  Sequencing Primer

<400>  87
agagtggagg cacaccgctt tgag                                               24


<210>  88
<211>  24
<212>  DNA
<213>  Artificial Sequence

<220>
<223>  Sequencing Primer

<400>  88
aggagtgaag ggatggcagc acag                                               24


<210>  89
<211>  26
<212>  DNA

<213>  Artificial Sequence

<220>
<223>  Sequencing Primer

<400>  89
tggctacttt tgttgccatc ctcatc                                            26


<210>  90
<211>  26
<212>  DNA
<213>  Artificial Sequence

<220>
<223>  Sequencing Primer

<400>  90
gcaaggtctc ccacagtgaa caatga                                            26


<210>  91
<211>  34
<212>  DNA
<213>  Artificial Sequence

<220>
<223>  Sequencing Primer

<400>  91
cacacataaa cccacataca gtttaacttc atcc                                   34


<210>  92
<211>  29
<212>  DNA
<213>  Artificial Sequence

<220>
<223>  Sequencing Primer

<400>  92
cactttctac tgttggctgg ttcagatca                                         29


<210>  93
<211>  28
<212>  DNA
<213>  Artificial Sequence

<220>
<223>  Sequencing Primer

<400>  93
tctacttttg cttcccctga cttcatcg                                          28


<210>  94
<211>  24
<212>  DNA
<213>  Artificial Sequence

<220>

<223>  Sequencing Primer

<400>  94
cacacttctg gcaccactcc cttc                                              24


<210>  95
<211>  24
<212>  DNA
<213>  Artificial Sequence

<220>
<223>  Sequencing Primer

<400>  95
tgggtctcca cacgtgttcc aatc                                              24


<210>  96
<211>  24
<212>  DNA
<213>  Artificial Sequence

<220>
<223>  Sequencing Primer

<400>  96
tggctgtctc cacagagatg ttgc                                              24


<210>  97
<211>  27
<212>  DNA
<213>  Artificial Sequence

<220>
<223>  Sequencing Primer

<400>  97
gaagaacttc cagaggagga gggagga                                           27


<210>  98
<211>  24
<212>  DNA
<213>  Artificial Sequence

<220>
<223>  Sequencing Primer

<400>  98
tggtgtgatc atgggctgga aaca                                              24


<210>  99
<211>  24
<212>  DNA
<213>  Artificial Sequence

<220>
<223>  Sequencing Primer

<400>  99

tccagcccat cccccatatt ccta                                              24

<210>  100
<211>  25
<212>  DNA
<213>  Artificial Sequence

<220>
<223>  Sequencing Primer

<400>  100
tgtccagcag tcgatgtgga tagca                                             25

<210>  101
<211>  28
<212>  DNA
<213>  Artificial Sequence

<220>
<223>  Sequencing Primer

<400>  101
cgttgcctta agtctctgtg tgaggaca                                          28

<210>  102
<211>  23
<212>  DNA
<213>  Artificial Sequence

<220>
<223>  Sequencing Primer

<400>  102
ccagattggg cccactgctt cct                                               23

<210>  103
<211>  24
<212>  DNA
<213>  Artificial Sequence

<220>
<223>  Sequencing Primer

<400>  103
tgggtagctg ggggtaacac gatg                                              24

<210>  104
<211>  24
<212>  DNA
<213>  Artificial Sequence

<220>
<223>  Sequencing Primer

<400>  104
gagcacagga aagggagggt ccag                                              24

<210>  105
<211>  32
<212>  DNA
<213>  Artificial Sequence

<220>
<223>  Sequencing Primer

<400>  105
catctgcttt ccttcaccct tagctaccta tt                                    32


<210>  106
<211>  24
<212>  DNA
<213>  Artificial Sequence

<220>
<223>  Sequencing Primer

<400>  106
gagcacaggc ttggtcttgc tgct                                             24


<210>  107
<211>  24
<212>  DNA
<213>  Artificial Sequence

<220>
<223>  Sequencing Primer

<400>  107
caaggccaca atcgaggttt tcca                                             24


<210>  108
<211>  24
<212>  DNA
<213>  Artificial Sequence

<220>
<223>  Sequencing Primer

<400>  108
gctggcatct ccacgcatct gata                                             24


<210>  109
<211>  21
<212>  DNA
<213>  Artificial Sequence

<220>
<223>  Sequencing Primer

<400>  109
agccttgcct ggctcccctg t                                                21


<210>  110
<211>  24
<212>  DNA

<213>  Artificial Sequence

<220>
<223>  Sequencing Primer

<400>  110
gaacccaggt gctgaccctt ctcc                                              24


<210>  111
<211>  24
<212>  DNA
<213>  Artificial Sequence

<220>
<223>  Sequencing Primer

<400>  111
tgctaaactg cccacctcag tcca                                              24


<210>  112
<211>  24
<212>  DNA
<213>  Artificial Sequence

<220>
<223>  Sequencing Primer

<400>  112
cgggcctgcc tcttctcctt tcta                                              24


<210>  113
<211>  24
<212>  DNA
<213>  Artificial Sequence

<220>
<223>  Sequencing Primer

<400>  113
tggaagttga ctcccaaccc acag                                              24


<210>  114
<211>  26
<212>  DNA
<213>  Artificial Sequence

<220>
<223>  Sequencing Primer

<400>  114
gcacctggct gtagagggag gtaagg                                            26


<210>  115
<211>  24
<212>  DNA
<213>  Artificial Sequence

<220>

<223>  Sequencing Primer

<400>  115
ccagcagccc ttcttatcgc tggt                                              24


<210>  116
<211>  38
<212>  DNA
<213>  Artificial Sequence

<220>
<223>  Sequencing Primer

<400>  116
gaaagtactg aagtttccaa gtacagagta acccctta                               38


<210>  117
<211>  24
<212>  DNA
<213>  Artificial Sequence

<220>
<223>  Sequencing Primer

<400>  117
attggctgct gtgggatgga aaca                                              24


<210>  118
<211>  27
<212>  DNA
<213>  Artificial Sequence

<220>
<223>  Sequencing Primer

<400>  118
ccagccctac cctcttcctt cttctcc                                           27


<210>  119
<211>  24
<212>  DNA
<213>  Artificial Sequence

<220>
<223>  Sequencing Primer

<400>  119
ctggcctttg tccctgagcc atct                                              24


<210>  120
<211>  24
<212>  DNA
<213>  Artificial Sequence

<220>
<223>  Sequencing Primer

<400>  120

atgtcgacag tgccgctgat gatg                                                24

<210>  121
<211>  20
<212>  DNA
<213>  Artificial Sequence

<220>
<223>  Sequencing Primer

<400>  121
cgcagcaccc agcagaccac                                                      20

<210>  122
<211>  24
<212>  DNA
<213>  Artificial Sequence

<220>
<223>  Sequencing Primer

<400>  122
   ggcatgggag tagggggagc ttct                                                24

<210>  123
<211>  25
<212>  DNA
<213>  Artificial Sequence

<220>
<223>  Sequencing Primer

<400>  123
gcttgtgtcc tctgctgagg ccttt                                                25

<210>  124
<211>  24
<212>  DNA
<213>  Artificial Sequence

<220>
<223>  Sequencing Primer

<400>  124
gcacgcttgt tttcctccat gcta                                                 24

<210>  125
<211>  29
<212>  DNA
<213>  Artificial Sequence

<220>
<223>  Sequencing Primer

<400>  125
gctcttcact actgtgttgg acatgctga                                            29

<210>  126
<211>  24
<212>  DNA
<213>  Artificial Sequence

<220>
<223>  Sequencing Primer

<400>  126
tcctgaggct ggacagcaga ttcc                                              24


<210>  127
<211>  24
<212>  DNA
<213>  Artificial Sequence

<220>
<223>  Sequencing Primer

<400>  127
ccaggttctg tgctgtcccc tgag                                              24


<210>  128
<211>  24
<212>  DNA
<213>  Artificial Sequence

<220>
<223>  Sequencing Primer

<400>  128
tgatgacatc tcgggtctgc ttgg                                              24


<210>  129
<211>  21
<212>  DNA
<213>  Artificial Sequence

<220>
<223>  Sequencing Primer

<400>  129
agttggggga gcgccagtca g                                                 21


<210>  130
<211>  24
<212>  DNA
<213>  Artificial Sequence

<220>
<223>  Sequencing Primer

<400>  130
aagccagcaa tcttgttgcc cttg                                              24


<210>  131
<211>  24
<212>  DNA

<213>  Artificial Sequence

<220>
<223>  Sequencing Primer

<400>  131
actgcccaag cagacccgag atgt                                              24


<210>  132
<211>  25
<212>  DNA
<213>  Artificial Sequence

<220>
<223>  Sequencing Primer

<400>  132
tgggctcctg aatctgatga gtctg                                             25


<210>  133
<211>  34
<212>  DNA
<213>  Artificial Sequence

<220>
<223>  Sequencing Primer

<400>  133
tgttttctaa gtcccaacag cttattgttt ttca                                   34


<210>  134
<211>  23
<212>  DNA
<213>  Artificial Sequence

<220>
<223>  Sequencing Primer

<400>  134
gctgctcctc tcctcgagcc act                                               23


<210>  135
<211>  24
<212>  DNA
<213>  Artificial Sequence

<220>
<223>  Sequencing Primer

<400>  135
   caccactctc ttggggctgg tttg                                               24


<210>  136
<211>  23
<212>  DNA
<213>  Artificial Sequence

<220>

<223>  Sequencing Primer

<400>  136
catcttctgg gggcagtggc agt                                              23


<210>  137
<211>  24
<212>  DNA
<213>  Artificial Sequence

<220>
<223>  Sequencing Primer

<400>  137
gcggttgctg ctctaccaca caca                                             24


<210>  138
<211>  25
<212>  DNA
<213>  Artificial Sequence

<220>
<223>  Sequencing Primer

<400>  138
tggtggactt cttcttgcgt tcctc                                            25


<210>  139
<211>  26
<212>  DNA
<213>  Artificial Sequence

<220>
<223>  Sequencing Primer

<400>  139
ctagagcctg agaaaaaggc tccaga                                           26


<210>  140
<211>  25
<212>  DNA
<213>  Artificial Sequence

<220>
<223>  Sequencing Primer

<400>  140
gagaggctgt gggagaactg tcacg                                            25


<210>  141
<211>  24
<212>  DNA
<213>  Artificial Sequence

<220>
<223>  Sequencing Primer

<400>  141

tccccgaccc cattcagcta caac                                              24


<210>  142
<211>  24
<212>  DNA
<213>  Artificial Sequence

<220>
<223>  Sequencing Primer

<400>  142
agaaccaggg tttgggtggt gcag                                              24


<210>  143
<211>  24
<212>  DNA
<213>  Artificial Sequence

<220>
<223>  Sequencing Primer

<400>  143
ggagcggccc ttatggtgtg acag                                              24


<210>  144
<211>  24
<212>  DNA
<213>  Artificial Sequence

<220>
<223>  Sequencing Primer

<400>  144
gggcaggggg agtgtacagg tagg                                              24


<210>  145
<211>  26
<212>  DNA
<213>  Artificial Sequence

<220>
<223>  Sequencing Primer

<400>  145
gagggatgag gagcctagag gtcagc                                            26


<210>  146
<211>  24
<212>  DNA
<213>  Artificial Sequence

<220>
<223>  Sequencing Primer

<400>  146
ttgctgctgc cggtacacag aggt                                              24

<210>  147
<211>  24
<212>  DNA
<213>  Artificial Sequence

<220>
<223>  Sequencing Primer

<400>  147
ttaccctgga gtgctgccca caac                                              24


<210>  148
<211>  24
<212>  DNA
<213>  Artificial Sequence

<220>
<223>  Sequencing Primer

<400>  148
cctgtgaccc tcaaaagccc ctct                                              24


<210>  149
<211>  25
<212>  DNA
<213>  Artificial Sequence

<220>
<223>  Sequencing Primer

<400>  149
tggcagcaag catttcctga gtttg                                             25


<210>  150
<211>  22
<212>  DNA
<213>  Artificial Sequence

<220>
<223>  Sequencing Primer

<400>  150
gcagcttgcc tcatccccct ag                                                22


<210>  151
<211>  24
<212>  DNA
<213>  Artificial Sequence

<220>
<223>  Sequencing Primer

<400>  151
tgagactagg gggatgaggc aagc                                              24


<210>  152
<211>  24
<212>  DNA

<213>  Artificial Sequence

<220>
<223>  Sequencing Primer

<400>  152
ttggtagaag ggtgggtgct ctgg                                              24


<210>  153
<211>  28
<212>  DNA
<213>  Artificial Sequence

<220>
<223>  Sequencing Primer

<400>  153
tccttatgtt tctcatgtgg gattgcag                                          28


<210>  154
<211>  24
<212>  DNA
<213>  Artificial Sequence

<220>
<223>  Sequencing Primer

<400>  154
tgagtggagg tcagtccatg tcca                                              24


<210>  155
<211>  24
<212>  DNA
<213>  Artificial Sequence

<220>
<223>  Sequencing Primer

<400>  155
tagatcctac ccgccacctg caac                                              24


<210>  156
<211>  24
<212>  DNA
<213>  Artificial Sequence

<220>
<223>  Sequencing Primer

<400>  156
agtcccacca ctttcaggca cagg                                              24


<210>  157
<211>  24
<212>  DNA
<213>  Artificial Sequence

<220>

<223>  Sequencing Primer

<400>  157
tctcctcacc tccctcatgc cttg                                              24


<210>  158
<211>  24
<212>  DNA
<213>  Artificial Sequence

<220>
<223>  Sequencing Primer

<400>  158
tgctgctgct gctcaggtag gatg                                              24


<210>  159
<211>  18
<212>  DNA
<213>  Artificial Sequence

<220>
<223>  Sequencing Primer

<400>  159
gagtgcccag ggcgtcca                                                     18


<210>  160
<211>  24
<212>  DNA
<213>  Artificial Sequence

<220>
<223>  Sequencing Primer

<400>  160
atcccagtgc cttacccgca ggat                                              24


<210>  161
<211>  24
<212>  DNA
<213>  Artificial Sequence

<220>
<223>  Sequencing Primer

<400>  161
agccatccta cctgagcagc agca                                              24


<210>  162
<211>  27
<212>  DNA
<213>  Artificial Sequence

<220>
<223>  Sequencing Primer

<400>  162

tgggaagaac ttgtgcctct cttcctc                                          27

<210>  163
<211>  24
<212>  DNA
<213>  Artificial Sequence

<220>
<223>  Sequencing Primer

<400>  163
atcccctgcc ctcagccctt tagt                                             24

<210>  164
<211>  22
<212>  DNA
<213>  Artificial Sequence

<220>
<223>  Sequencing Primer

<400>  164
aggagccgcc tgttgctgct gt                                               22

<210>  165
<211>  34
<212>  DNA
<213>  Artificial Sequence

<220>
<223>  Sequencing Primer

<400>  165
tgaagtatct tttgtgttct tatagcagca gcag                                  34

<210>  166
<211>  24
<212>  DNA
<213>  Artificial Sequence

<220>
<223>  Sequencing Primer

<400>  166
gtccctgagc ctggggctgt agtc                                             24

<210>  167
<211>  31
<212>  DNA
<213>  Artificial Sequence

<220>
<223>  Sequencing Primer

<400>  167
aaaaacggat tgggaaagga ggttgaagaa g                                     31

<210>  168
<211>  24
<212>  DNA
<213>  Artificial Sequence

<220>
<223>  Sequencing Primer

<400>  168
ttccatgggg ttctccttgg gaag                                              24


<210>  169
<211>  24
<212>  DNA
<213>  Artificial Sequence

<220>
<223>  Sequencing Primer

<400>  169
atacccacac ccctgcctgg tctt                                              24


<210>  170
<211>  24
<212>  DNA
<213>  Artificial Sequence

<220>
<223>  Sequencing Primer

<400>  170
gggccccaac cactactggt gcta                                              24


<210>  171
<211>  19
<212>  DNA
<213>  Artificial Sequence

<220>
<223>  Sequencing Primer

<400>  171
atacgggcgg cgggagctg                                                    19


<210>  172
<211>  21
<212>  DNA
<213>  Artificial Sequence

<220>
<223>  Sequencing Primer

<400>  172
ccggcgccgt aggtttctca c                                                 21


<210>  173
<211>  24
<212>  DNA

<213>  Artificial Sequence

<220>
<223>  Sequencing Primer

<400>  173
cttgtcacca ggggcagctg ctat                                              24


<210>  174
<211>  27
<212>  DNA
<213>  Artificial Sequence

<220>
<223>  Sequencing Primer

<400>  174
ttcaggaaaa catggctctc catatcc                                           27


<210>  175
<211>  24
<212>  DNA
<213>  Artificial Sequence

<220>
<223>  Sequencing Primer

<400>  175
atgccatgag gaaagctggt gtgg                                              24


<210>  176
<211>  24
<212>  DNA
<213>  Artificial Sequence

<220>
<223>  Sequencing Primer

<400>  176
ccccacccct acaccctagg acac                                              24


<210>  177
<211>  24
<212>  DNA
<213>  Artificial Sequence

<220>
<223>  Sequencing Primer

<400>  177
gggagtccga tgactgcgct tctt                                              24


<210>  178
<211>  28
<212>  DNA
<213>  Artificial Sequence

<220>

<223>  Sequencing Primer

<400>  178
cagagatctc aacaacccag aaatgcaa                                          28


<210>  179
<211>  24
<212>  DNA
<213>  Artificial Sequence

<220>
<223>  Sequencing Primer

<400>  179
tggggattaa ggcttgggca aatg                                              24


<210>  180
<211>  25
<212>  DNA
<213>  Artificial Sequence

<220>
<223>  Sequencing Primer

<400>  180
ccacctcaaa tccaaagttg ggaga                                             25


<210>  181
<211>  26
<212>  DNA
<213>  Artificial Sequence

<220>
<223>  Sequencing Primer

<400>  181
ggtaggccct ttcctgagaa gctctg                                            26


<210>  182
<211>  20
<212>  DNA
<213>  Artificial Sequence

<220>
<223>  Sequencing Primer

<400>  182
tggctgtccc atggcaccaa                                                   20


<210>  183
<211>  29
<212>  DNA
<213>  Artificial Sequence

<220>
<223>  Sequencing Primer

<400>  183

ccacagatcc tatgaatgca ctccagagc 29

<210> 184
<211> 21
<212> DNA
<213> Artificial Sequence

<220>
<223> Sequencing Primer

<400> 184
acagccatgc tgtgaggggc c 21

<210> 185
<211> 20
<212> DNA
<213> Artificial Sequence

<220>
<223> Sequencing Primer

<400> 185
ttggtgccat gggacagcca 20

<210> 186
<211> 30
<212> DNA
<213> Artificial Sequence

<220>
<223> Sequencing Primer

<400> 186
gcttttcttg ataccatcat aagcacagga 30

<210> 187
<211> 23
<212> DNA
<213> Artificial Sequence

<220>
<223> Sequencing Primer

<400> 187
tttctgggaa tctgccctgt gga 23

<210> 188
<211> 26
<212> DNA
<213> Artificial Sequence

<220>
<223> Sequencing Primer

<400> 188
tgctgcacta ctgcttggaa ctgctg 26

<210>  189
<211>  20
<212>  DNA
<213>  Artificial Sequence

<220>
<223>  Sequencing Primer

<400>  189
agcagcaggc ggcgctacag                                                    20


<210>  190
<211>  31
<212>  DNA
<213>  Artificial Sequence

<220>
<223>  Sequencing Primer

<400>  190
tgatgatgca atttaattag atgctgctgc t                                       31


<210>  191
<211>  24
<212>  DNA
<213>  Artificial Sequence

<220>
<223>  Sequencing Primer

<400>  191
gcagtccaag cagtagtgca gcag                                               24


<210>  192
<211>  21
<212>  DNA
<213>  Artificial Sequence

<220>
<223>  Sequencing Primer

<400>  192
gggcactgca cccagcattg t                                                  21


<210>  193
<211>  24
<212>  DNA
<213>  Artificial Sequence

<220>
<223>  Sequencing Primer

<400>  193
ccagactgtc acagggagga gctg                                               24


<210>  194
<211>  23
<212>  DNA

<213>  Artificial Sequence

<220>
<223>  Sequencing Primer

<400>  194
aattggtggc tgggcctgca aag                                               23


<210>  195
<211>  23
<212>  DNA
<213>  Artificial Sequence

<220>
<223>  Sequencing Primer

<400>  195
aacagcagct gcagcgaata gca                                               23


<210>  196
<211>  23
<212>  DNA
<213>  Artificial Sequence

<220>
<223>  Sequencing Primer

<400>  196
gctggtggtg ctgtgtgtga tgc                                               23


<210>  197
<211>  21
<212>  DNA
<213>  Artificial Sequence

<220>
<223>  Sequencing Primer

<400>  197
caggcccagc caccaattca g                                                 21


<210>  198
<211>  24
<212>  DNA
<213>  Artificial Sequence

<220>
<223>  Sequencing Primer

<400>  198
gttctgagca actggaggct gctg                                              24


<210>  199
<211>  24
<212>  DNA
<213>  Artificial Sequence

<220>

<223>  Sequencing Primer

<400>  199
agcagcctcc agttgctcag aacc                                              24


<210>  200
<211>  21
<212>  DNA
<213>  Artificial Sequence

<220>
<223>  Sequencing Primer

<400>  200
acgggctgca tgtgctccac t                                                 21


<210>  201
<211>  29
<212>  DNA
<213>  Artificial Sequence

<220>
<223>  Sequencing Primer

<400>  201
acaggaagac taggctctgc tctctgacc                                         29


<210>  202
<211>  24
<212>  DNA
<213>  Artificial Sequence

<220>
<223>  Sequencing Primer

<400>  202
ggcagcctga gctgtctgta ctgc                                              24


<210>  203
<211>  20
<212>  DNA
<213>  Artificial Sequence

<220>
<223>  Sequencing Primer

<400>  203
gtggtgcagc agcccccagt                                                   20


<210>  204
<211>  24
<212>  DNA
<213>  Artificial Sequence

<220>
<223>  Sequencing Primer

<400>  204

ccagccccta atccaaccag aacc                                                24

<210>  205
<211>  24
<212>  DNA
<213>  Artificial Sequence

<220>
<223>  Sequencing Primer

<400>  205
aggctacacg tgccctctcc acct                                                24

<210>  206
<211>  24
<212>  DNA
<213>  Artificial Sequence

<220>
<223>  Sequencing Primer

<400>  206
aaagggatgg agcttgacgg gatg                                                24

<210>  207
<211>  24
<212>  DNA
<213>  Artificial Sequence

<220>
<223>  Sequencing Primer

<400>  207
gcccaaggtg ggatgcacat aaga                                                24

<210>  208
<211>  24
<212>  DNA
<213>  Artificial Sequence

<220>
<223>  Sequencing Primer

<400>  208
gtgcgcttta caaaggcgct caga                                                24

<210>  209
<211>  21
<212>  DNA
<213>  Artificial Sequence

<220>
<223>  Sequencing Primer

<400>  209
ggcaggtggt gtggaggcag g                                                   21

<210>  210
<211>  21
<212>  DNA
<213>  Artificial Sequence

<220>
<223>  Sequencing Primer

<400>  210
tggagttggg ctgtgagctg g                                                21


<210>  211
<211>  20
<212>  DNA
<213>  Artificial Sequence

<220>
<223>  Sequencing Primer

<400>  211
gcagaccccg cagtcgatgc                                                  20


<210>  212
<211>  24
<212>  DNA
<213>  Artificial Sequence

<220>
<223>  Sequencing Primer

<400>  212
agaaacatgt gagccggggt ccag                                             24


<210>  213
<211>  25
<212>  DNA
<213>  Artificial Sequence

<220>
<223>  Sequencing Primer

<400>  213
gaccccggct cacatgtttc tctca                                            25


<210>  214
<211>  27
<212>  DNA
<213>  Artificial Sequence

<220>
<223>  Sequencing Primer

<400>  214
caggtgtgtt taaaggtcca ggtgagg                                          27


<210>  215
<211>  22
<212>  DNA

<213>  Artificial Sequence

<220>
<223>  Sequencing Primer

<400>  215
agccctccca gagcccagtg ac                                                22


<210>  216
<211>  24
<212>  DNA
<213>  Artificial Sequence

<220>
<223>  Sequencing Primer

<400>  216
ccagagctcg cacacccagt atga                                              24


<210>  217
<211>  24
<212>  DNA
<213>  Artificial Sequence

<220>
<223>  Sequencing Primer

<400>  217
tcatactggg tgtgcgagct ctgg                                              24


<210>  218
<211>  26
<212>  DNA
<213>  Artificial Sequence

<220>
<223>  Sequencing Primer

<400>  218
cagctgcttc agcttgtcca ggtact                                            26


<210>  219
<211>  25
<212>  DNA
<213>  Artificial Sequence

<220>
<223>  Sequencing Primer

<400>  219
ctgcagtgaa ccccagctct gtcat                                             25


<210>  220
<211>  21
<212>  DNA
<213>  Artificial Sequence

<220>

<223>  Sequencing Primer

<400>  220
ggtggtgtcc ccagggtgca g                                                21


<210>  221
<211>  26
<212>  DNA
<213>  Artificial Sequence

<220>
<223>  Sequencing Primer

<400>  221
caccaccagg cttgtgtctt agtgtg                                           26


<210>  222
<211>  24
<212>  DNA
<213>  Artificial Sequence

<220>
<223>  Sequencing Primer

<400>  222
tgggctatct gtgcccttgt ggac                                             24


<210>  223
<211>  25
<212>  DNA
<213>  Artificial Sequence

<220>
<223>  Sequencing Primer

<400>  223
tgagcctgag aactgccatg tcttc                                            25


<210>  224
<211>  23
<212>  DNA
<213>  Artificial Sequence

<220>
<223>  Sequencing Primer

<400>  224
tggttcccct aggggtgtct ggc                                              23


<210>  225
<211>  22
<212>  DNA
<213>  Artificial Sequence

<220>
<223>  Sequencing Primer

<400>  225

accatgggcc tggggtgtga ag                                                22

<210>  226
<211>  25
<212>  DNA
<213>  Artificial Sequence

<220>
<223>  Sequencing Primer

<400>  226
gcggtacagg gaatggttga agaca                                             25

<210>  227
<211>  18
<212>  DNA
<213>  Artificial Sequence

<220>
<223>  Sequencing Primer

<400>  227
cagccgctcc tggatgcc                                                     18

<210>  228
<211>  22
<212>  DNA
<213>  Artificial Sequence

<220>
<223>  Sequencing Primer

<400>  228
ggtgaccaca cctggcacac gc                                                22

<210>  229
<211>  21
<212>  DNA
<213>  Artificial Sequence

<220>
<223>  Sequencing Primer

<400>  229
gccaggtgtg gtcaccatgc c                                                 21

<210>  230
<211>  27
<212>  DNA
<213>  Artificial Sequence

<220>
<223>  Sequencing Primer

<400>  230
agtgagaagg gtccaggttt accagga                                           27

<210>  231
<211>  23
<212>  DNA
<213>  Artificial Sequence

<220>
<223>  Sequencing Primer

<400>  231
cgcaggcttg aggatgatga gcg                                               23


<210>  232
<211>  23
<212>  DNA
<213>  Artificial Sequence

<220>
<223>  Sequencing Primer

<400>  232
agggcctgct ttcccacacc agt                                               23


<210>  233
<211>  27
<212>  DNA
<213>  Artificial Sequence

<220>
<223>  Sequencing Primer

<400>  233
ggggcttgtc caggtcacag atagagc                                           27


<210>  234
<211>  30
<212>  DNA
<213>  Artificial Sequence

<220>
<223>  Sequencing Primer

<400>  234
gacagctcct ctgggtctac ctaccgtact                                        30


<210>  235
<211>  26
<212>  DNA
<213>  Artificial Sequence

<220>
<223>  Sequencing Primer

<400>  235
caggacacat cacctcctgg tgcttc                                            26


<210>  236
<211>  21
<212>  DNA

<213>  Artificial Sequence

<220>
<223>  Sequencing Primer

<400>  236
ggcccaggtg ttgagcaagg c                                                21


<210>  237
<211>  22
<212>  DNA
<213>  Artificial Sequence

<220>
<223>  Sequencing Primer

<400>  237
catgacctcc aggctgctgc ag                                               22


<210>  238
<211>  31
<212>  DNA
<213>  Artificial Sequence

<220>
<223>  Sequencing Primer

<400>  238
agctaaccta accccaggct ctctaaggaa g                                     31


<210>  239
<211>  3384
<212>  DNA
<213>  Homo sapiens

<400>  239
ggtttgggcc tggctctgtg actgaggcgg cggcggtggc ggccaagcgg gatacgggcg      60

gcgggagctg gggaacaggc atggacgttt ccgggcaaga gaccgactgg cggagcaccg      120

ccttccggca gaagctggtc agtcaaatcg aggatgccat gaggaaagct ggtgtggcac      180

acagtaaatc cagcaaggat atggagagcc atgttttcct gaaggccaag acccgggacg      240

aatacctttc tctcgtggcc aggctcatta tccattttcg agacattcat aacaagaaat      300

ctcaagcttc cgtcagtgat cctatgaatg cactccagag cctgactggc ggacctgctg      360

cgggagccgc tggaattggc atgcctcctc ggggcccggg acagtctctg ggcgggatgg      420

gtagccttgg tgccatggga cagccaatgt ctctctcagg gcagccgcct cctgggacct      480

cggggatggc ccctcacagc atggctgtcg tgtctacggc aactccacag acccagctgc      540

agctccagca ggtggcgctg cagcagcagc agcaacagca gcagttccag cagcagcagc      600

aggcggcgct acagcagcag cagcagcagc agcaacagca gcagttccag gctcagcaga      660

gtgccatgca gcagcagttc caagcagtag tgcagcagca gcagcagctc cagcagcagc      720

agcagcagca gcagcatcta attaaattgc atcatcaaaa tcagcaacag atacagcagc 780

agcaacagca gctgcagcga atagcacagc tgcagctcca acaacagcaa cagcagcagc 840

agcagcagca gcagcagcag cagcaggctt tgcaggccca gccaccaatt cagcagccac 900

cgatgcagca gccacagcct ccgccctccc aggctctgcc ccagcagctg cagcagatgc 960

atcacacaca gcaccaccag ccgccaccac agccccagca gcctccagtt gctcagaacc 1020

aaccatcaca actcccgcca cagtcgcaga cccagccttt ggtgtcacag gcgcaagctc 1080

tccctggaca aatgttgtat acccaaccac cactgaaatt tgtccgagct ccgatggtgg 1140

tgcagcagcc cccagtgcag ccccaggtgc agcagcagca gacagcagta cagacagctc 1200

aggctgccca gatggtggct cccggagtcc agatgatcac ggaagccttg gcccaaggtg 1260

ggatgcacat aagagcccgg ttcccgccta ccaccgctgt gtccgccatc ccgtcaagct 1320

ccatcccttt gggcagacag cccatggcac aggtcagcca gagcagcctc cccatgctgt 1380

cctcgccgtc accgggccag caggtgcaga ccccgcagtc gatgccccct cccccccagc 1440

cgtccccgca gcccggccag cccagctcac agcccaactc caacgtcagc tctggccctg 1500

ccccatctcc cagtagcttc ctgcccagcc cctcaccgca gccctcccag agcccagtga 1560

cggcgcggac cccacagaac ttcagtgtcc cctcacctgg acctttaaac acacctgtga 1620

accccagctc tgtcatgagc ccagctggct ccagccaggc tgaggagcag cagtacctgg 1680

acaagctgaa gcagctgtcg aagtacatcg agcccctgcg ccgcatgatc aacaagatcg 1740

acaagaacga agacagaaaa aaggacctga gtaagatgaa gagccttctg gacattctga 1800

cagacccctc gaagcggtgt cccctgaaga ccttgcaaaa gtgtgagatc gccctggaga 1860

aactcaagaa tgacatggcg gtgcccactc ccccaccgcc cccggtgcca ccgaccaaac 1920

agcagtacct atgccagccg ctcctggatg ccgtcctggc caacatccgc tcacctgtct 1980

tcaaccattc cctgtaccgc acattcgttc cagccatgac cgccattcac ggcccaccca 2040

tcacggcccc agtggtgtgc acccggaagc gcaggcttga ggatgatgag cggcagagca 2100

tccccagtgt gctccagggt gaggtggcca ggctggaccc caagttcctg gtaaacctgg 2160

acccttctca ctgcagcaac aatggcactg tccacctgat ctgcaagctg gatgacaagg 2220

acctcccaag tgtgccacca ctggagctca gtgtgcccgc tgactatcct gcccaaagcc 2280

cgctgtggat agaccggcag tggcagtacg acgccaaccc cttcctccag tcggtgcacc 2340

gctgcatgac ctccaggctg ctgcagctcc cggacaagca ctcggtcacc gccttgctca 2400

acacctgggc ccagagcgtc caccaggcct gcctctcagc cgcctagcca agactgcagg 2460

gatggcccgc agcctcatcg gggccaagga cacacgcctc ctgtcagaca cttctaggtg 2520

ttggcttcct tagagagcct ggggttaggt tagctttcct gcttttatct tctgccttgg 2580

ggacctgcca aacgaaatcc cacacctgta cagaactggg ataggcgcag tggagcgggt 2640

tgcttggggg gcgttggccg acttcttaga gaaggccctc catgtgactt cctcccagga 2700

gccagatgcg atcctcaggc tgctctcacc gtggcctgtc cacggtccag gtccatctca 2760

gcagcgtgag ggtgcactca gggtgttgtt agagcgtctc gtgtgtgcta gacgcacccc 2820

tactcgttcc tatagaacac agaggacata ggaaaccctt aaaacacaca tgggattctc 2880

tggtcacagt tttgggttca ggctatgctg ctttgggcag gtggagcacc ccccgaggaa 2940

gcctgcaagt ccagggcaca ggctgccttt tggagggagg gctggcccat aggtgctgct 3000

ggctccccgc caccagctgg gcctcagccc tcacggcatt cctgctgagc accgtggggc 3060

acccagggag caggggcgtc agggatcctg ctgccggcac ccctgtgccg ctggcatgag 3120

ggccgtgtcc ccactgtgaa ggatgaagag caaggccctc aggacccgtg tcctcagagc 3180

accacacact gagcacccag agacagcggg cctggcagcg ggccgggcca tgcagggagc 3240

gcctccctat gttgcctgcc actctgggca ccggccagca ccctctggtg agaagaggtc 3300

cccccttttt atgtgcacta ccccaccatc tgtgattata ataaatttat tattcctgtg 3360

tttgtcagtt gttcaaaaaa aaaa 3384

<210> 240
<211> 788
<212> PRT
<213> Homo sapiens

<400> 240

Met Asp Val Ser Gly Gln Glu Thr Asp Trp Arg Ser Thr Ala Phe Arg
1               5                   10                  15

Gln Lys Leu Val Ser Gln Ile Glu Asp Ala Met Arg Lys Ala Gly Val
            20                  25                  30

Ala His Ser Lys Ser Ser Lys Asp Met Glu Ser His Val Phe Leu Lys
        35                  40                  45

Ala Lys Thr Arg Asp Glu Tyr Leu Ser Leu Val Ala Arg Leu Ile Ile
    50                  55                  60

His Phe Arg Asp Ile His Asn Lys Lys Ser Gln Ala Ser Val Ser Asp
65                  70                  75                  80

Pro Met Asn Ala Leu Gln Ser Leu Thr Gly Gly Pro Ala Ala Gly Ala
                85                  90                  95

Ala Gly Ile Gly Met Pro Pro Arg Gly Pro Gly Gln Ser Leu Gly Gly
            100                 105                 110

Met Gly Ser Leu Gly Ala Met Gly Gln Pro Met Ser Leu Ser Gly Gln
        115                 120                 125

Pro Pro Pro Gly Thr Ser Gly Met Ala Pro His Ser Met Ala Val Val
    130                 135                 140

Ser Thr Ala Thr Pro Gln Thr Gln Leu Gln Leu Gln Gln Val Ala Leu
145                 150                 155                 160

Gln Gln Gln Gln Gln Gln Gln Gln Phe Gln Gln Gln Gln Gln Ala Ala
                165                 170                 175

Leu Gln Gln Gln Gln Gln Gln Gln Gln Gln Gln Gln Phe Gln Ala Gln
            180                 185                 190

Gln Ser Ala Met Gln Gln Gln Phe Gln Ala Val Val Gln Gln Gln Gln
        195                 200                 205

Gln Leu Gln Gln Gln Gln Gln Gln Gln Gln His Leu Ile Lys Leu His
    210                 215                 220

His Gln Asn Gln Gln Gln Ile Gln Gln Gln Gln Gln Gln Leu Gln Arg
225                 230                 235                 240

Ile Ala Gln Leu Gln Leu Gln Gln Gln Gln Gln Gln Gln Gln Gln Gln
                245                 250                 255

Gln Gln Gln Gln Gln Gln Ala Leu Gln Ala Gln Pro Pro Ile Gln Gln
            260                 265                 270

Pro Pro Met Gln Gln Pro Gln Pro Pro Pro Ser Gln Ala Leu Pro Gln
        275                 280                 285

Gln Leu Gln Gln Met His His Thr Gln His His Gln Pro Pro Pro Gln
    290                 295                 300

Pro Gln Gln Pro Pro Val Ala Gln Asn Gln Pro Ser Gln Leu Pro Pro
305                 310                 315                 320

Gln Ser Gln Thr Gln Pro Leu Val Ser Gln Ala Gln Ala Leu Pro Gly
                325                 330                 335

Gln Met Leu Tyr Thr Gln Pro Pro Leu Lys Phe Val Arg Ala Pro Met
            340                 345                 350

Val Val Gln Gln Pro Pro Val Gln Pro Gln Val Gln Gln Gln Gln Thr
        355                 360                 365

Ala Val Gln Thr Ala Gln Ala Ala Gln Met Val Ala Pro Gly Val Gln
    370                 375                 380

Met Ile Thr Glu Ala Leu Ala Gln Gly Gly Met His Ile Arg Ala Arg
385                 390                 395                 400

Phe Pro Pro Thr Thr Ala Val Ser Ala Ile Pro Ser Ser Ser Ile Pro
                405                 410                 415

Leu Gly Arg Gln Pro Met Ala Gln Val Ser Gln Ser Ser Leu Pro Met
            420                 425                 430

Leu Ser Ser Pro Ser Pro Gly Gln Gln Val Gln Thr Pro Gln Ser Met
        435                 440                 445

Pro Pro Pro Pro Gln Pro Ser Pro Gln Pro Gly Gln Pro Ser Ser Gln
    450                 455                 460

Pro Asn Ser Asn Val Ser Ser Gly Pro Ala Pro Ser Pro Ser Ser Phe
465                 470                 475                 480

Leu Pro Ser Pro Ser Pro Gln Pro Ser Gln Ser Pro Val Thr Ala Arg
                485                 490                 495

Thr Pro Gln Asn Phe Ser Val Pro Ser Pro Gly Pro Leu Asn Thr Pro
            500                 505                 510

Val Asn Pro Ser Ser Val Met Ser Pro Ala Gly Ser Ser Gln Ala Glu
        515                 520                 525

Glu Gln Gln Tyr Leu Asp Lys Leu Lys Gln Leu Ser Lys Tyr Ile Glu
    530                 535                 540

Pro Leu Arg Arg Met Ile Asn Lys Ile Asp Lys Asn Glu Asp Arg Lys
545                 550                 555                 560

Lys Asp Leu Ser Lys Met Lys Ser Leu Leu Asp Ile Leu Thr Asp Pro
                565                 570                 575

Ser Lys Arg Cys Pro Leu Lys Thr Leu Gln Lys Cys Glu Ile Ala Leu
            580                 585                 590

Glu Lys Leu Lys Asn Asp Met Ala Val Pro Thr Pro Pro Pro Pro Pro
        595                 600                 605

Val Pro Pro Thr Lys Gln Gln Tyr Leu Cys Gln Pro Leu Leu Asp Ala
    610                 615                 620

Val Leu Ala Asn Ile Arg Ser Pro Val Phe Asn His Ser Leu Tyr Arg
625                 630                 635                 640

Thr Phe Val Pro Ala Met Thr Ala Ile His Gly Pro Pro Ile Thr Ala
                645                 650                 655

Pro Val Val Cys Thr Arg Lys Arg Arg Leu Glu Asp Asp Glu Arg Gln
            660                 665                 670

Ser Ile Pro Ser Val Leu Gln Gly Glu Val Ala Arg Leu Asp Pro Lys
        675                 680                 685

Phe Leu Val Asn Leu Asp Pro Ser His Cys Ser Asn Asn Gly Thr Val
    690                 695                 700

His Leu Ile Cys Lys Leu Asp Asp Lys Asp Leu Pro Ser Val Pro Pro
705                 710                 715                 720

Leu Glu Leu Ser Val Pro Ala Asp Tyr Pro Ala Gln Ser Pro Leu Trp
                725                 730                 735

Ile Asp Arg Gln Trp Gln Tyr Asp Ala Asn Pro Phe Leu Gln Ser Val
            740                 745                 750

His Arg Cys Met Thr Ser Arg Leu Leu Gln Leu Pro Asp Lys His Ser
        755                 760                 765

Val Thr Ala Leu Leu Asn Thr Trp Ala Gln Ser Val His Gln Ala Cys
    770                 775                 780

Leu Ser Ala Ala
785

<210>  241
<211>  10
<212>  DNA
<213>  Artificial Sequence

<220>
<223>  cDNA human MED15 (isoform)

<400>  241
cccccccccc                                                            10

<210>  242
<211>  748
<212>  PRT
<213>  Homo sapiens

<400>  242
   Met Asp Val Ser Gly Gln Glu Thr Asp Trp Arg Ser Thr Ala Phe Arg
   1               5                   10                  15
   Gln Lys Leu Val Ser Gln Ile Glu Asp Ala Met Arg Lys Ala Gly Val
               20                  25                  30
   Ala His Ser Lys Ser Ser Lys Asp Met Glu Ser His Val Phe Leu Lys
           35                  40                  45
   Ala Lys Thr Arg Asp Glu Tyr Leu Ser Leu Val Ala Arg Leu Ile Ile
       50                  55                  60
   His Phe Arg Asp Ile His Asn Lys Lys Ser Gln Ala Ser Val Ser Asp
   65                  70                  75                  80
   Pro Met Asn Ala Leu Gln Ser Leu Thr Gly Gly Pro Ala Ala Gly Ala
                   85                  90                  95
   Ala Gly Ile Gly Met Pro Pro Arg Gly Pro Gly Gln Ser Leu Gly Gly
               100                 105                 110
   Met Gly Ser Leu Gly Ala Met Gly Gln Pro Met Ser Leu Ser Gly Gln
           115                 120                 125
   Pro Pro Pro Gly Thr Ser Gly Met Ala Pro His Ser Met Ala Val Val
       130                 135                 140
   Ser Thr Ala Thr Pro Gln Thr Gln Leu Gln Leu Gln Gln Val Ala Leu
   145                 150                 155                 160
   Gln Gln Gln Gln Gln Gln Gln Gln Phe Gln Gln Gln Gln Gln Ala Ala
                   165                 170                 175
   Leu Gln Gln Gln Gln Gln Gln Gln Gln Gln Gln Gln Phe Gln Ala Gln
               180                 185                 190
   Gln Ser Ala Met Gln Gln Gln Phe Gln Ala Val Val Gln Gln Gln Gln
           195                 200                 205
   Gln Leu Gln Gln Gln Gln Gln Gln Gln Gln His Leu Ile Lys Leu His
       210                 215                 220
   His Gln Asn Gln Gln Gln Ile Gln Gln Gln Gln Gln Gln Leu Gln Arg
   225                 230                 235                 240
   Ile Ala Gln Leu Gln Leu Gln Gln Gln Gln Gln Gln Gln Gln Gln Gln
   245 250 255
   Gln Gln Gln Gln Gln Gln Ala Leu Gln Ala Gln Pro Pro Ile Gln Gln
   260 265 270
   Pro Pro Met Gln Gln Pro Gln Pro Pro Pro Ser Gln Ala Leu Pro Gln
   275 280 285
   Gln Leu Gln Gln Met His His Thr Gln His His Gln Pro Pro Pro Gln
   290 295 300
   Pro Gln Gln Pro Pro Val Ala Gln Asn Gln Pro Ser Gln Leu Pro Pro
   305 310 315 320
   Gln Ser Gln Thr Gln Pro Leu Val Ser Gln Ala Gln Ala Leu Pro Gly
   325 330 335
   Gln Met Leu Tyr Thr Gln Pro Pro Leu Lys Phe Val Arg Ala Pro Met
   340 345 350
   Val Val Gln Gln Pro Pro Val Gln Pro Gln Val Gln Gln Gln Gln Thr
   355 360 365
   Ala Val Gln Thr Ala Gln Ala Ala Gln Met Val Ala Pro Gly Val Gln
   370 375 380
   Val Ser Gln Ser Ser Leu Pro Met Leu Ser Ser Pro Ser Pro Gly Gln
   385 390 395 400
   Gln Val Gln Thr Pro Gln Ser Met Pro Pro Pro Pro Gln Pro Ser Pro
   405 410 415
   Gln Pro Gly Gln Pro Ser Ser Gln Pro Asn Ser Asn Val Ser Ser Gly
   420 425 430
   Pro Ala Pro Ser Pro Ser Ser Phe Leu Pro Ser Pro Ser Pro Gln Pro
   435 440 445
   Ser Gln Ser Pro Val Thr Ala Arg Thr Pro Gln Asn Phe Ser Val Pro
   450 455 460
   Ser Pro Gly Pro Leu Asn Thr Pro Val Asn Pro Ser Ser Val Met Ser
   465 470 475 480
   Pro Ala Gly Ser Ser Gln Ala Glu Glu Gln Gln Tyr Leu Asp Lys Leu
   485 490 495
   Lys Gln Leu Ser Lys Tyr Ile Glu Pro Leu Arg Arg Met Ile Asn Lys
               500                 505                 510
   Ile Asp Lys Asn Glu Asp Arg Lys Lys Asp Leu Ser Lys Met Lys Ser
           515                 520                 525
   Leu Leu Asp Ile Leu Thr Asp Pro Ser Lys Arg Cys Pro Leu Lys Thr
       530                 535                 540
   Leu Gln Lys Cys Glu Ile Ala Leu Glu Lys Leu Lys Asn Asp Met Ala
   545                 550                 555                 560
   Val Pro Thr Pro Pro Pro Pro Pro Val Pro Pro Thr Lys Gln Gln Tyr
                   565                 570                 575
   Leu Cys Gln Pro Leu Leu Asp Ala Val Leu Ala Asn Ile Arg Ser Pro
               580                 585                 590
   Val Phe Asn His Ser Leu Tyr Arg Thr Phe Val Pro Ala Met Thr Ala
           595                 600                 605
   Ile His Gly Pro Pro Ile Thr Ala Pro Val Val Cys Thr Arg Lys Arg
       610                 615                 620
   Arg Leu Glu Asp Asp Glu Arg Gln Ser Ile Pro Ser Val Leu Gln Gly
   625                 630                 635                 640
   Glu Val Ala Arg Leu Asp Pro Lys Phe Leu Val Asn Leu Asp Pro Ser
                   645                 650                 655
   His Cys Ser Asn Asn Gly Thr Val His Leu Ile Cys Lys Leu Asp Asp
               660                 665                 670
   Lys Asp Leu Pro Ser Val Pro Pro Leu Glu Leu Ser Val Pro Ala Asp
           675                 680                 685
   Tyr Pro Ala Gln Ser Pro Leu Trp Ile Asp Arg Gln Trp Gln Tyr Asp
       690                 695                 700
   Ala Asn Pro Phe Leu Gln Ser Val His Arg Cys Met Thr Ser Arg Leu
   705                 710                 715                 720
   Leu Gln Leu Pro Asp Lys His Ser Val Thr Ala Leu Leu Asn Thr Trp
                   725                 730                 735
   Ala Gln Ser Val His Gln Ala Cys Leu Ser Ala Ala
               740                 745

<210>  243
<211>  10
<212>  DNA
<213>  Artificial Sequence

<220>
<223>  cDNA human MED15 (isoform)

<400>  243
cccccccccc                                                              10


<210>  244
<211>  6985
<212>  DNA
<213>  Homo sapiens

<400>  244
attgtccgat ggttcccggc gtacctcggc ttccctcggt agtttccggc aatggtcgag        60

agtttctaac gtgccccctt gttgtctctc ggccgccgtc ctctcaacca ccgcccccct        120

tttcggctcc ctctccccct tcccgttccc ccagtcagcc tggccctgct ggtgcctccg       180

gcgctacggg ctgggcaaga tggcggcctt cgggatcttg agctacgaac accggcccct       240

gaagcggccg cggctggggc ctcccgatgt ttaccctcag gaccccaaac agaaggagga       300

tgaactgacg gccttgaatg taaaacaagg tttcaataac cagcctgctg tctctgggga       360

tgagcatggc agtgccaaga acgtcagctt caatcctgcc aagatcagtt ccaacttcag       420

cagcattatt gcagagaaat tacgttgtaa tacccttcct gacactggtc gcaggaagcc       480

ccaagtgaac cagaaggata acttctggct ggtgactgca cgatcccaga gtgccattaa       540

cacttggttc actgacttgg ctggcaccaa gccactcacg caactagcca aaaaggtccc       600

cattttcagt aagaaggaag aggtgtttgg gtacttagcc aaatacacag tgcctgtgat       660

gcgggctgcc tggctcatta agatgacctg tgcctactat gcagcaatct ctgagaccaa       720

ggttaagaag agacatgttg accctttcat ggaatggact cagatcatca ccaagtactt       780

atgggagcag ttacagaaga tggctgaata ctaccggcca gggcctgcag gaagtggggg       840

ctgtggttcc acgatagggc ccttgcccca tgatgtagag gtggcaatcc ggcagtggga       900

ttacaccgag aagctggcca tgttcatgtt tcaggatgga atgctggaca gacatgagtt       960

cctgacctgg gtgcttgagt gttttgagaa gatccgccct ggagaggatg aattgcttaa      1020

actgctgctg cctctgcttc tccgatactc tggggaattt gttcagtctg catacctgtc      1080

ccgccggctt gcctacttct gtacacggag actggccctg cagctggatg gtgtgagcag      1140

tcactcatct catgttatat ctgctcagtc aacaagcacg ctacccacca cccctgctcc      1200

tcagccccca actagcagca caccctcgac tccctttagt gacctgctta tgtgccctca      1260

gcaccggccc ctggtttttg gcctcagctg tatcctacag accatcctcc tgtgctgtcc      1320

tagtgccttg gtttggcact actcactgac tgatagcaga attaagaccg gctcaccact 1380

tgaccacttg cctattgccc cgtccaacct gcccatgcca gagggtaaca gtgccttcac 1440

tcagcaggtc cgtgcaaagt tgcgggagat cgagcagcag atcaaggagc ggggacaggc 1500

agttgaagtt cgctggtctt tcgataaatg ccaggaagct actgcaggct tcaccattgg 1560

acgggtactt catactttgg aagtgctgga cagccatagt tttgaacgct ctgacttcag 1620

caactctctt gactcccttt gtaaccgaat ctttggattg ggacctagca aggatgggca 1680

tgagatctcc tcagatgatg atgctgtggt gtcattgcta tgtgaatggg ctgtcagctg 1740

caagcgttct ggtcggcatc gtgctatggt ggtagccaag ctcctggaga agagacaggc 1800

ggagattgag gctgagcgtt gtggagaatc agaagccgca gatgagaagg gttccatcgc 1860

ctctggctcc ctttctgctc ccagtgctcc cattttccag gatgtcctcc tgcagtttct 1920

ggatacacag gctcccatgc tgacggaccc tcgaagtgag agtgagcggg tggaattctt 1980

taacttagta ctgctgttct gtgaactgat tcgacatgat gttttctccc acaacatgta 2040

tacttgcact ctcatctccc gaggggacct tgcctttgga gcccctggtc cccggcctcc 2100

ctctcccttt gatgatcctg ccgatgaccc agagcacaag gaggctgaag gcagcagcag 2160

cagcaagctg gaagatccag ggctctcaga atctatggac attgacccta gttccagtgt 2220

tctctttgag gacatggaga agcctgattt ctcattgttc tcccctacta tgccctgtga 2280

ggggaagggc agtccatccc ctgagaagcc agatgtcgag aaggaggtga agcccccacc 2340

caaggagaag attgaaggga cccttggggt tctttacgac cagccacgac acgtgcagta 2400

cgccacccat tttcccatcc cccaggagga gtcatgcagc catgagtgca accagcggtt 2460

ggtcgtactg tttggggtgg gaaagcagcg agatgatgcc cgccatgcca tcaagaaaat 2520

caccaaggat atcttgaagg ttctgaaccg caaagggaca gcagaaactg accagcttgc 2580

tcctattgtg cctctgaatc ctggagacct gacattctta ggtggggagg atgggcagaa 2640

gcggcgacgc aaccggcctg aagccttccc cactgctgaa gatatctttg ctaagttcca 2700

gcacctttca cattatgacc aacaccaggt cacggctcag gtctcccgga atgttctgga 2760

gcagatcacg agctttgccc ttggcatgtc ataccacttg cctctggtgc agcatgtgca 2820

gttcatcttc gacctcatgg aatattcact cagcatcagt ggcctcatcg actttgccat 2880

tcagctgctg aatgaactga gtgtagttga ggctgagctg cttctcaaat cctcggatct 2940

ggtgggcagc tacactacta gcctgtgcct gtgcatcgtg gctgtcctgc ggcactatca 3000

tgcctgcctc atcctcaacc aggaccagat ggcacaggtc tttgaggggc tgtgtggcgt 3060

cgtgaagcat gggatgaacc ggtccgatgg ctcctctgca gagcgctgta tccttgctta 3120

tctctatgat ctgtacacct cctgtagcca tttaaagaac aaatttgggg agctcttcag 3180

cgacttttgc tcaaaggtga agaacaccat ctactgcaac gtggagccat cggaatcaaa 3240

tatgcgctgg gcacctgagt tcatgatcga cactctagag aaccctgcag ctcacacctt 3300

cacctacacg gggctaggca agagtcttag tgagaaccct gctaaccgct acagctttgt 3360

ctgcaatgcc cttatgcacg tctgtgtggg gcaccatgat cccgataggg tgaatgacat 3420

cgcaatcctg tgtgcagagc tgaccggcta ttgcaagtca ctgagtgcag aatggctagg 3480

agtgcttaag gccttgtgct gctcctctaa caatggcact tgtggtttca acgatctcct 3540

ctgcaatgtt gatgtcagtg acctatcttt tcatgactcg ctggctactt ttgttgccat 3600

cctcatcgct cggcagtgtt tgctcctgga agatctgatt cgctgtgctg ccatcccttc 3660

actccttaat gctgcttgta gtgaacagga ctctgagcca ggggcccggc ttacctgccg 3720

catcctcctt caccttttca agacaccgca gctcaatcct tgccagtctg atggaaacaa 3780

gcctacagta ggaatccgct cctcctgcga ccgccacctg ctggctgcct cccagaaccg 3840

catcgtggat ggagccgtgt ttgctgttct caaggctgtg tttgtacttg gggatgcgga 3900

actgaaaggt tcaggcttca ctgtgacagg aggaacagaa gaacttccag aggaggaggg 3960

aggaggtggc agtggtggtc ggaggcaggg tggccgcaac atctctgtgg agacagccag 4020

tctggatgtc tatgccaagt acgtgctgcg cagcatctgc caacaggaat gggtaggaga 4080

acgttgcctt aagtctctgt gtgaggacag caatgacctg caagacccag tgttgagtag 4140

tgcccaggcg cagcgcctca tgcagctcat ttgctatcca catcgactgc tggacaatga 4200

ggatggggaa aacccccagc ggcagcgcat aaagcgcatt ctccagaact tggaccagtg 4260

gaccatgcgc cagtcttcct tggagctgca gctcatgatc aagcagaccc ctaacaatga 4320

gatgaactcc ctcttggaga acatcgccaa ggccacaatc gaggttttcc aacagtcagc 4380

agagacaggg tcatcttctg gaagtactgc aagcaacatg cccagcagca gcaagaccaa 4440

gcctgtgctc agctctctag agcgctctgg tgtatggctg gtggcccccc tcattgctaa 4500

actgcccacc tcagtccagg gacatgtgtt aaaggctgct ggggaagaat tggagaaggg 4560

tcagcacctg ggttcctctt cacgcaaaga acgtgatcga caaaagcaga agagcatgtc 4620

cctattgagc cagcagccct tcttatcgct ggtgctaaca tgtctgaaag ggcaggatga 4680

acaacgcgag ggactcctta cctccctcta cagccaggtg caccagattg tgaataattg 4740

gcgagatgac cagtacttag atgattgcaa accaaagcag cttatgcatg aggcactcaa 4800

actgcggctc aacctggtgg ggggcatgtt tgacacggtg cagcgcagca cccagcagac 4860

cacggagtgg gccatgctcc tcctggagat catcatcagc ggcactgtcg acatgcagtc 4920

caacaatgag ctcttcacta ctgtgttgga catgctgagc gtgctcatca atgggacatt 4980

ggctgcagac atgtctagca tctcgcaagg tagcatggag gaaaacaagc gtgcatacat 5040

gaacctggcg aagaagttgc agaaggagtt gggggagcgc cagtcagaca gtctggaaaa 5100

ggttcgccag ctgctgccac tgcccaagca gacccgagat gtcatcacgt gtgagccaca 5160

gggctccctt atcgatacca agggcaacaa gattgctggc ttcgattcca tcttcaagaa 5220

ggagggtcta caggtttcca ccaaacagaa gatctcgccc tgggatcttt ttgaggggtt 5280

gaagccgtca gcaccactct cttggggctg gtttggaaca gtccgagtgg accggcgagt 5340

ggctcgagga gaggagcagc agcggttgct gctctaccac acacacctga ggccccggcc 5400

ccgcgcctat tacctggagc cactgccact gcccccagaa gatgaggagc cgcctgctcc 5460

taccctgcta gagcctgaga aaaaggctcc agagcccccc aaaactgaca aaccgggggc 5520

tgctccaccc agtactgagg aacgcaagaa gaagtccacc aagggcaaga aacgcagcca 5580

gccagctacc aagacagagg actatggaat gggcccgggt cggagcggcc cttatggtgt 5640

gacagtgcct ccggacctcc tgcaccaccc aaaccctggt tctataacac accttaacta 5700

caggcaaggc tccataggcc tgtacaccca gaaccagcca ctacctgcag gtggccctcg 5760

tgtggaccca taccgtcctg tgcgcttacc aatgcagaag ctgcccaccc gaccaactta 5820

ccctggagtg ctgcccacaa ccatgactgg cgtcatgggt ttagaaccct cctcttataa 5880

gacctctgtg taccggcagc agcaacctgc ggtgccccaa ggacagcgcc ttcgccaaca 5940

gctccagcag agtcagggca tgttgggaca gtcatctgtc catcagatga ctcccagctc 6000

ttcctacggt ttgcagactt cccagggcta tactccttat gtttctcatg tgggattgca 6060

gcaacacaca ggccctgcag gtaccatggt gccccccagc tactccagcc agccttacca 6120

gagcacccac ccttctacca atcctactct tgtagatcct acccgccacc tgcaacagcg 6180

gcccagtggc tatgtgcacc agcaggcccc cacctatgga catggactga cctccactca 6240

aaggttttca caccagacac tgcagcagac acccatgata agtaccatga ctccaatgag 6300

tgcccagggc gtccaggcag gcgtccgttc aacagccatc ctacctgagc agcagcagca 6360

gcagcaacag cagcaacagc aacagcagca gcagcagcaa cagcaacagc agcagcagca 6420

gcagcagtac cacatccggc agcagcagca gcagcagatc ctgcggcagc agcagcaaca 6480

gcaacagcag cagcagcagc agcagcaaca gcaacagcag cagcagcaac agcaacaaca 6540

gcaacaccag cagcaacagc agcaacaggc ggctcctccc caaccccagc cccagtccca 6600

gccccagttc cagcgccagg ggcttcagca gacccagcag cagcaacaga cagcagcttt 6660

ggtccggcaa cttcaacaac agctctctaa tacccagcca cagcccagta ccaacatatt 6720

tggacgctac tgagccacct ggaggaactg cttgtgcact ggatgtggcc ccaccctttc 6780

ctcttaattc ccaatcccat tcctgggcta gcaccagtag tggttggggc cctcccctca 6840

ggctccattt ttaataagtt tttagtattt ttgttaatgt gaggcattga gctgttgggt 6900

tttgtatatt atttatatag agaccccaga gctgttgcac ccaatacaca gagcttcttt 6960

gcaaagggaa aaaaaaaaaa aaaaa 6985

<210>  245
<211>  2177
<212>  PRT
<213>  Homo sapiens

<400>  245

<210>  246
<211>  11
<212>  DNA
<213>  Artificial Sequence

<220>
<223>  cDNA human MED12

<400>  246
cccccccccc c                                                            11

## Description

### Field of the invention

The present invention relates to methods of diagnosing the risk of developing cancer or determining the prognosis of a patient diagnosed with a cancer, using mediator 15 (MED15) and/or mediator 12 (MED12) as markers. Furthermore, the invention relates to methods of identifying compounds for treatment of cancer and to said compounds.

### Background

Cancer is a class of diseases which occurs because cells become immortalized; they fail to heed customary signals to turn off growth which is a normal function of remodeling in the body that requires cells to die on cue. Apoptosis, or programmed cell death, can become defective and when this happens malignant transformation can take place. The immortalized cells grow beyond their normal limits and invade adjacent tissues. The malignant cells may also metastasize and spread to other locations in the body via the bloodstream or lymphatic system. Cancer cells often form a mass known as a tumor.

There are about 200 different types of cancer; the cancers can start in any type of body tissue although many cancers will metastasize into other body tissues. There are many different causes of cancer and these include; carcinogens, age, genetic mutations, immune system problems, diet, weight, lifestyle, environmental factors such as pollutants, some viruses (for example the human papilloma virus (HPV) is implicated in cervical cancer) and some bacterial infections are also known to cause cancers.

There are many different treatment options for cancer and the treatment sought is often determined by the type and stage of the cancer. Treatment options include; chemotherapeutic drug treatment, hormonal drug treatment, radiotherapy, surgery, complementary therapies and combinations thereof.

One example of cancer is prostate cancer. Prostate cancer is the most common type of cancer in men and accounts for 24% of all UK male cancers. In 2006 there were over 35,000 new cases of prostate cancer diagnosed in the UK alone.

The prostate is a gland in the male reproductive system and symptoms of cancer in the prostate can include pain, difficulty urinating, problems with sexual intercourse and erectile dysfunction. Prostate cancer may metastasize to the bones and or lymph nodes. Treatment options for prostate cancer include surgery, radiation therapy, chemotherapy, and hormone treatment.

Hormone treatment usually involves treatment with an anti-androgen such as cyproterone acetate, flutamide or bicalutamide, either alone or in combination with a chemotherapeutic agent. These treatments work to stop the production of testosterone (androgen) which can slow down tumor growth or even shrink the tumor. While the prostate cancer cells are responding to anti-androgens, they are referred to as "hormone-sensitive" or "androgen-sensitive" prostate cancer. Unfortunately, after a few years of treatment with anti-androgens the prostate cancer stops responding to hormone treatment and is termed "hormone-insensitive" or "castration-resistant" prostate cancer. At this stage the cancer growth cannot be controlled by the hormone treatment.

Afflicting one out of nine men over age 65, prostate cancer (PCA) is a leading cause of male cancer-related death, second only to lung cancer (Abate-Shen and Shen, Genes Dev 14:2410 (2000); Ruijter et al., Endocr Rev, 20:22 (1999)). Thus, there is a need in the field for a reliable diagnostic test and treatment of cancer, in particular prostate cancer.

### Summary of the Invention

The present invention relates to a method of diagnosing the risk of developing cancer or determining the prognosis of a patient suffering from cancer, said method comprising:
I.
   (a) determining the level of mediator 15 (MED15) and/or mediator 12 (MED12) in a sample obtained from a patient,
   (b) comparing the data obtained in step a) to a predetermined level of MED15 and/or MED12 observed in a healthy individual, wherein when the level of MED15 and/or MED 12 is increased compared to the predetermined levels in a healthy individual said patient is diagnosed with an increased risk to develop cancer or an unfavorable prognosis, or
II.
   (a) detecting the presence or absence of at least one mutation in the MED15 and/or the MED12 gene in a sample obtained from a patient, wherein said at least one mutation is selected from a group consisting of reference SNP numbers rs117046295, rs361923, rs165643, rs165835, rs6209586 and rs5030619 or a c 1965-10 G>A mutation within the MED15 gene, wherein when at least one of said mutations is detected, said patient is diagnosed with an increased risk to develop cancer or an unfavorable prognosis.

In a further aspect, the present invention relates to the use of MED15 and/or MED12 for diagnosis of cancer or prognosis of a patient suffering from cancer.

In a third aspect, the present invention relates to a method of identifying a compound for treatment of cancer, said method comprising (a) contacting cancer cells in culture with a potential cancer modulating compound; (b) determining the level of MED15 and/or MED12 in said cancer cells, and (c) comparing the data obtained in step b) to a predetermined level of MED15 and/or MED12 observed in non-cancer cells or in said cancer cells before the contacting step (a), wherein a decrease in the level of MED15 and/or MED12 indicates that said compound may be useful for the treatment of cancer.

In specific embodiments of the invention the cancer is prostate cancer, preferably castration-resistant prostate cancer (CRPC) and more preferably distant metastatic CRPC or local recurrent CRPC.

In a further aspect, the present invention relates to the use of MED15 and/or MED12 to identify a compound for treatment of cancer, wherein the cancer is prostate cancer, preferably castration-resistant prostate cancer (CRPC) and more preferably distant metastatic CRPC or local recurrent CRPC.

The present invention is further directed to compounds inhibiting MED15 and/or MED12 gene expression or the activity of MED15 and/or MED12 gene products for use in the treatment of cancer, wherein the cancer is prostate cancer, preferably castration-resistant prostate cancer (CRPC) and more preferably distant metastatic CRPC or local recurrent CRPC.

### Brief Description of the Drawings

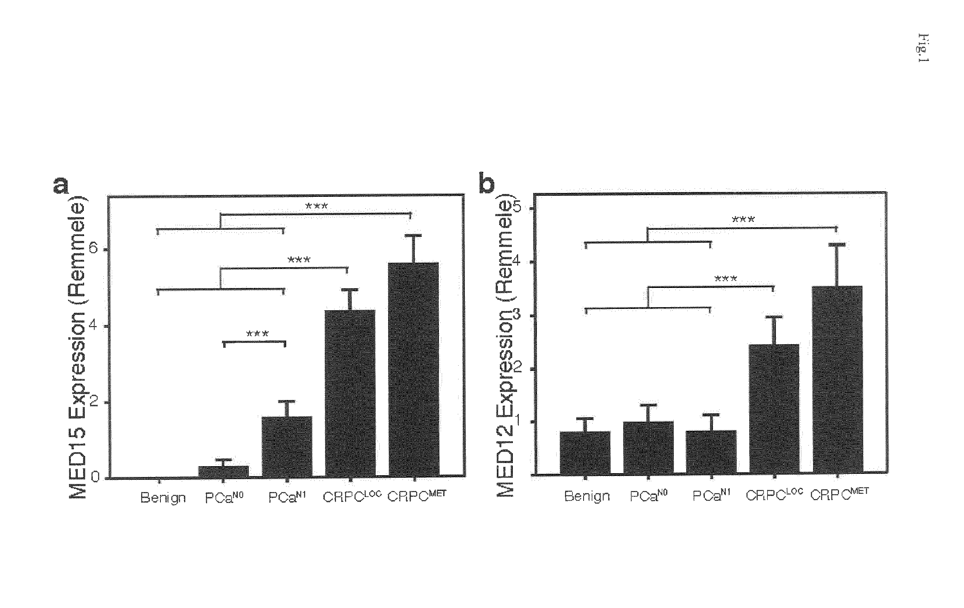

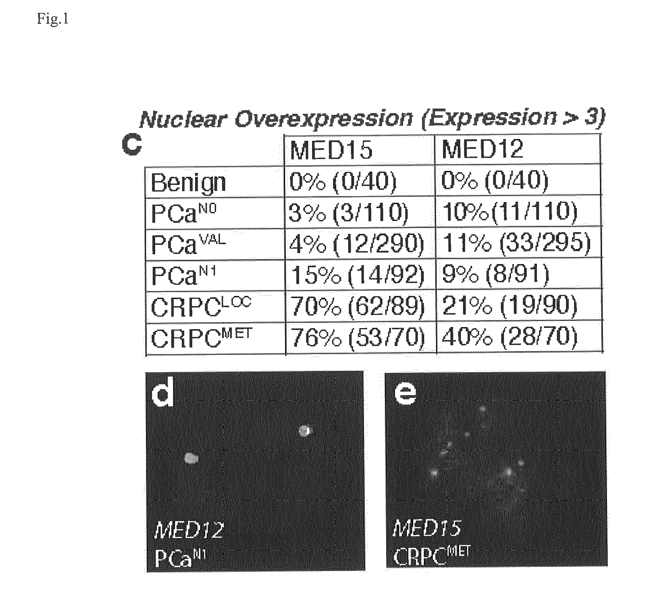

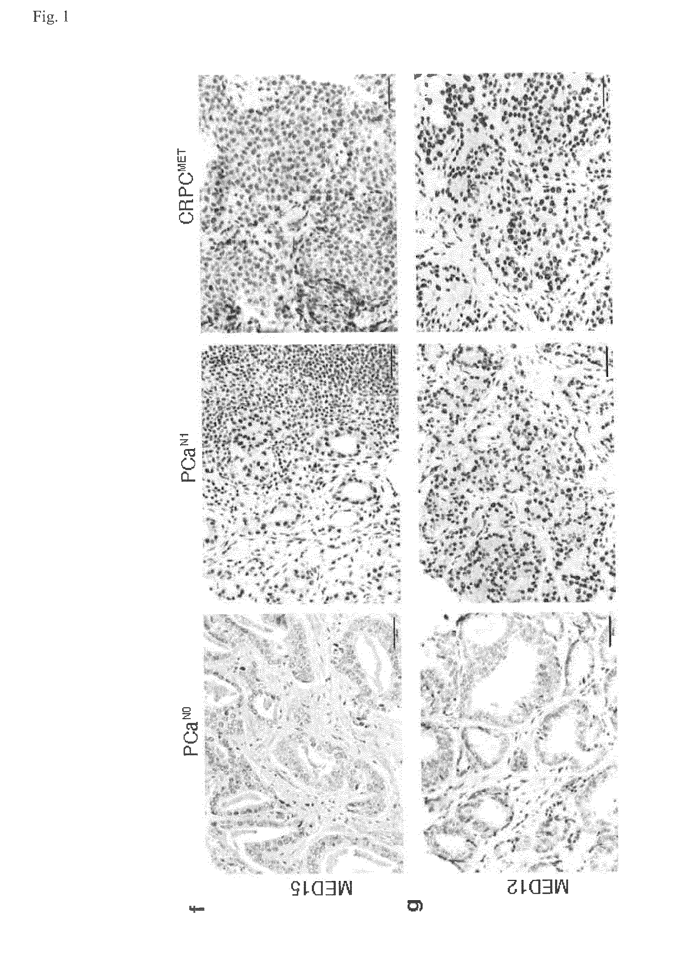

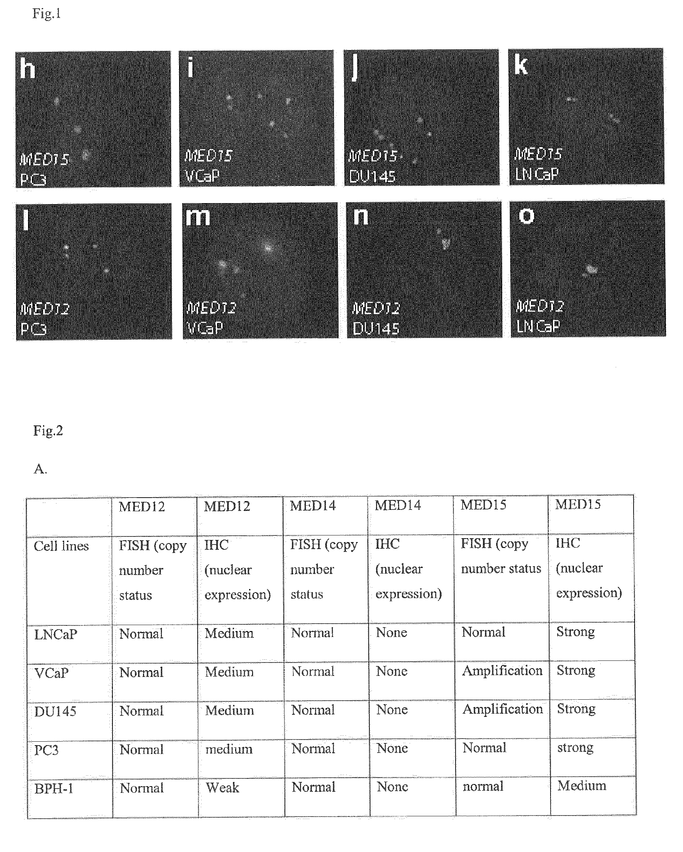

Figure 1: MED15 and MED12 expression in prostate cancer. a) and b) MED15 and MED12 nuclear expression is higher in distant metastatic castration-resistant prostate cancer (CRPC^{LOC}) and local recurrent castration-resistant prostate cancer (CRPC^{MET}), as compared to androgen-sensitive prostate cancer (PCa). *** P < 0.001, Kruskal-Wallis test (ANOVA). c) Table summarizing the percentage of cases with MED15 and MED12 nuclear overexpression. d) and e) Representative FISH images, showing a deletion of MED12 in PCa^{N1}(d), and an amplification of MED15 in CRPC^{MET}(e). f) Immunhistochemical staining of MED15 and (g) MED12 in PCa^{N0} and CRPC^{MET}. Scale bar, 20µm. h-o) Representative FISH images, showing a normal MED15 level in PC3 (h) and LNCaP (k), a normal MED12 level in PC3 (1), VCaP (m), DU145 (n), and LNCaP (o), and a MED15 amplification in VCaP (i) and DU145 (j).
Figure 2: Genetic phenotypes in cancer patients and nuclear expression of MED12, MED14 and MED15. The Table summarizes the percentage of all assessable cases with normal appearance, amplification and deletion of MED12, MED14, and MED15.

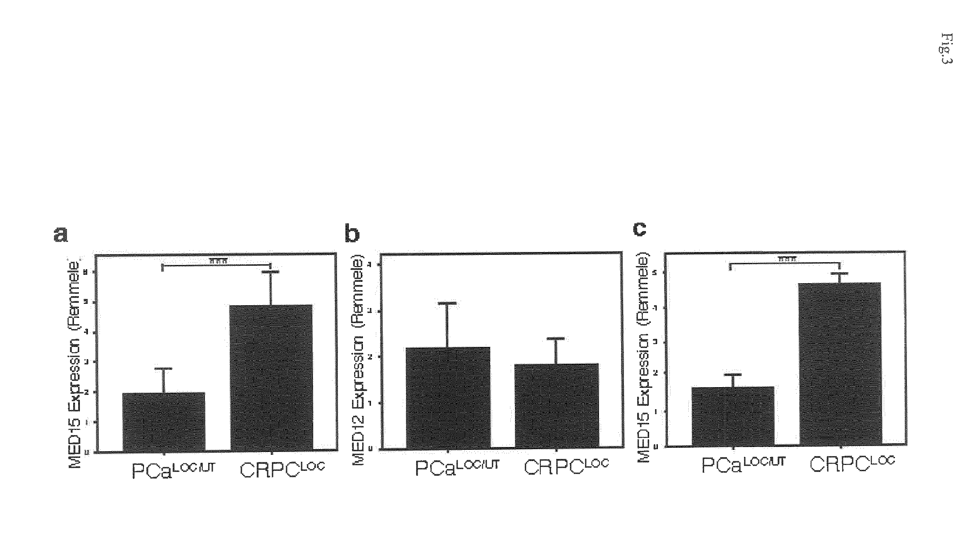

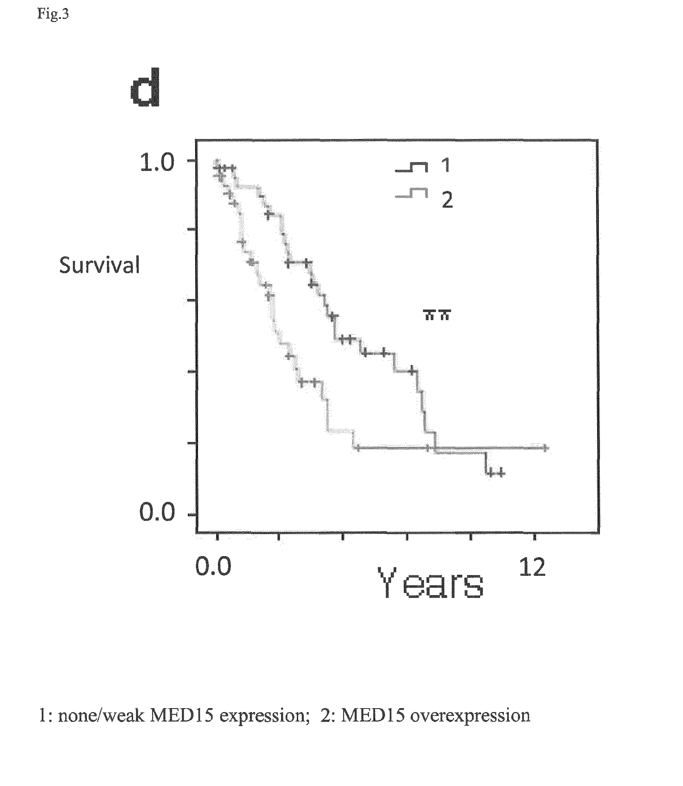

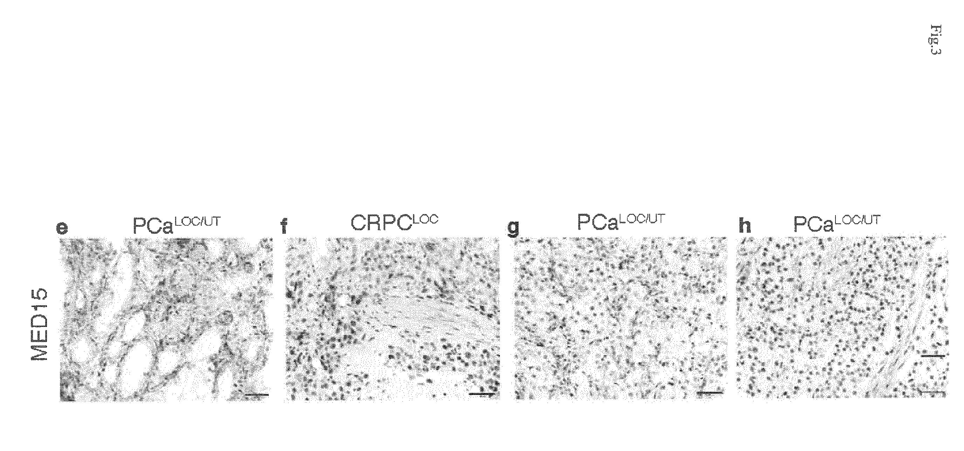

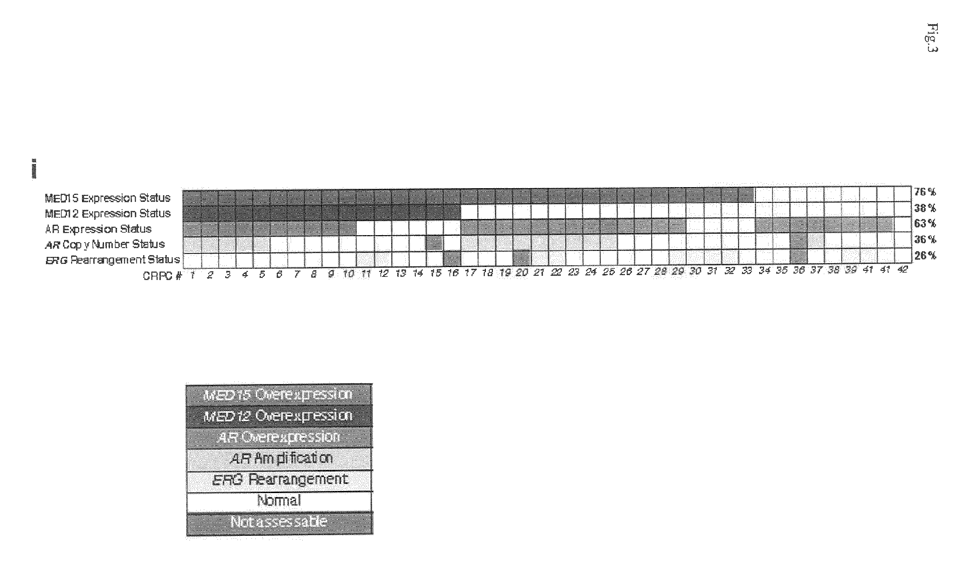

Figure 3: MED15 expression is increased following anti-androgen therapy. a) and b) MED15 (a) and MED12 (b) expression in hormone-naive untreated locally recurrent PCa (PCa^{LOC/UT}) and corresponding CRPC^{LOC}. In 65% of cases, following anti-androgen therapy, MED15 expression was higher in CRPC^{LOC}. *** P < 0.001, Wilcoxon signed-rank test. c) MED15 expression of all patients with PCa^{LOC/UT} and CRPC^{LOC}. *** P < 0.001, Man-Whitney test. d) Kaplan-Meier estimates the effect of MED15 nuclear overexpression on survival in patients with PCa^{LOC/UT}. ** P < 0.02, log-rank test. e-h) Immunohistochemical staining of PCa^{LOC/UT} and CRPC^{LOC} for MED15. The figure shows corresponding PCa^{LOC/UT} and CRPC^{LOC} before (e) and after (f) anti-androgen therapy. PCa^{LOC/UT} with weak (g) and high (h) MED15 expression are depicted. Scale bar, 20µm. i) Heatmap representation of MED15, MED12, androgen receptor (AR) expression, AR copy number status, and ETS-related gene (ERG) rearrangement in a series of 42 CRPC^{MET}.

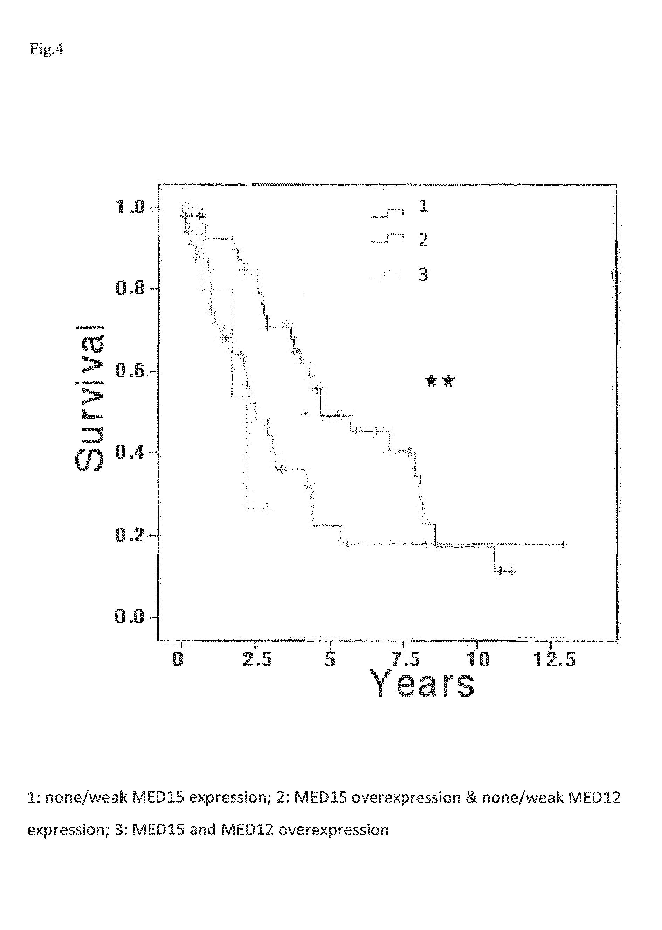

Figure 4: Prognosis of cancer patients overexpressing MED15 and MED12. Kaplan-Meier estimates the effect of MED15 and MED12 nuclear overexpression on survival in patients with PCa^{LOC/UT}. ** P < 0.03, log-rank test.

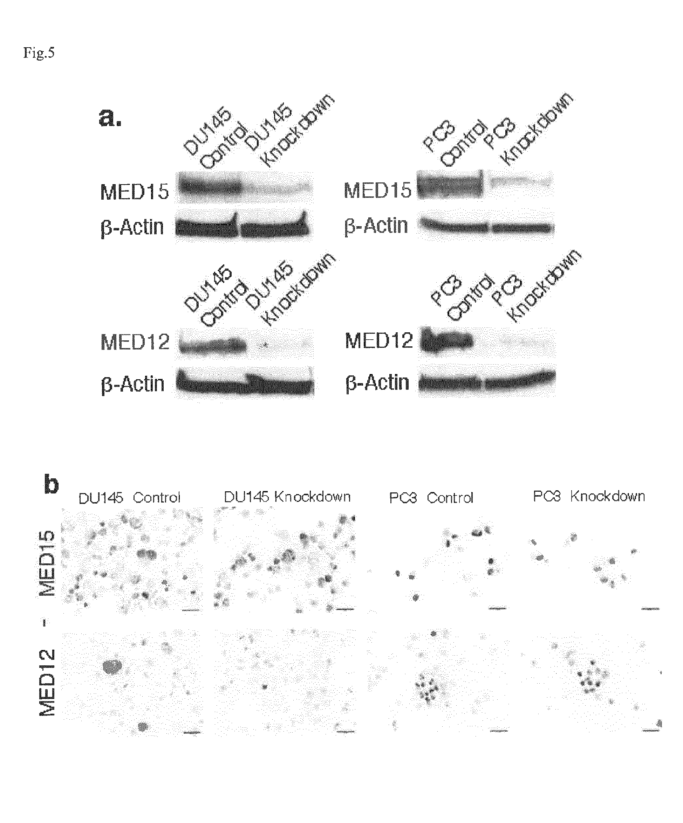

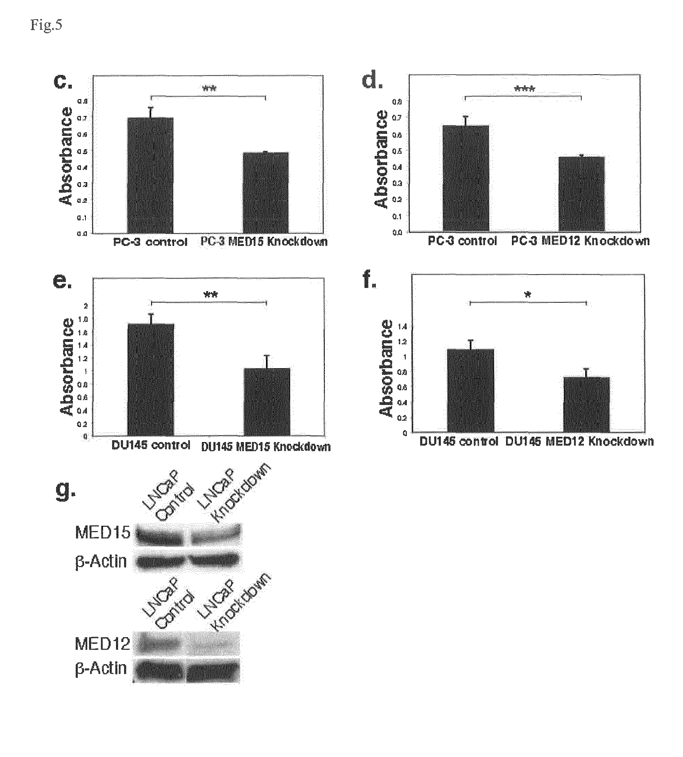

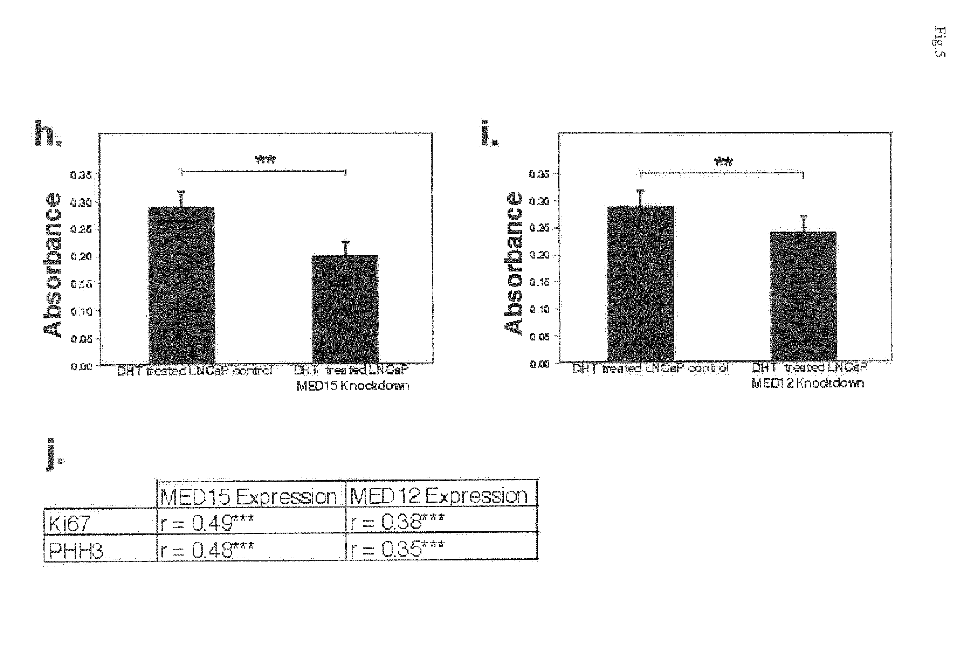

Figure 5: MED15 and MED12 knockdown in PCa cell lines. a) and b) siRNA mediated MED15 and MED12 knockdown in DU145 and PC3 cell lines. c) and d) MED15 and MED12 knockdown in PC3 cells exhibited a significant decrease in proliferation compared to control. * P < 0.05, ** P < 0.01, *** P < 0.001. e) and f) Similarly as in c) and d), MED15 and MED12 knockdown in the DU145 cells decreased proliferation. * P < 0.05, ** P < 0.01, *** P < 0.001. g) siRNA mediated MED15 and MED12 knockdown in LNCaP cell line. h) and i) MED15 and MED12 knockdown in the LNCaP cells treated with dihydrotestosterone (DHT) decreased proliferation. * P < 0.05, ** P < 0.01, *** P < 0.001. j) Correlation of MED15 and MED12 expression and tumor proliferation (Ki67, PHH3). *** P < 0.001, Pearson.

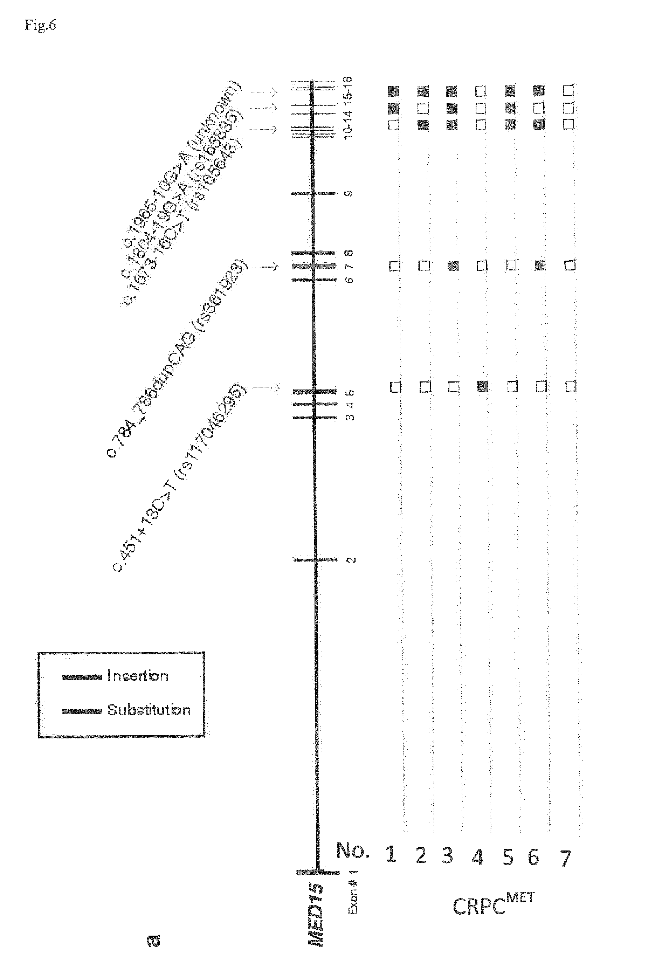

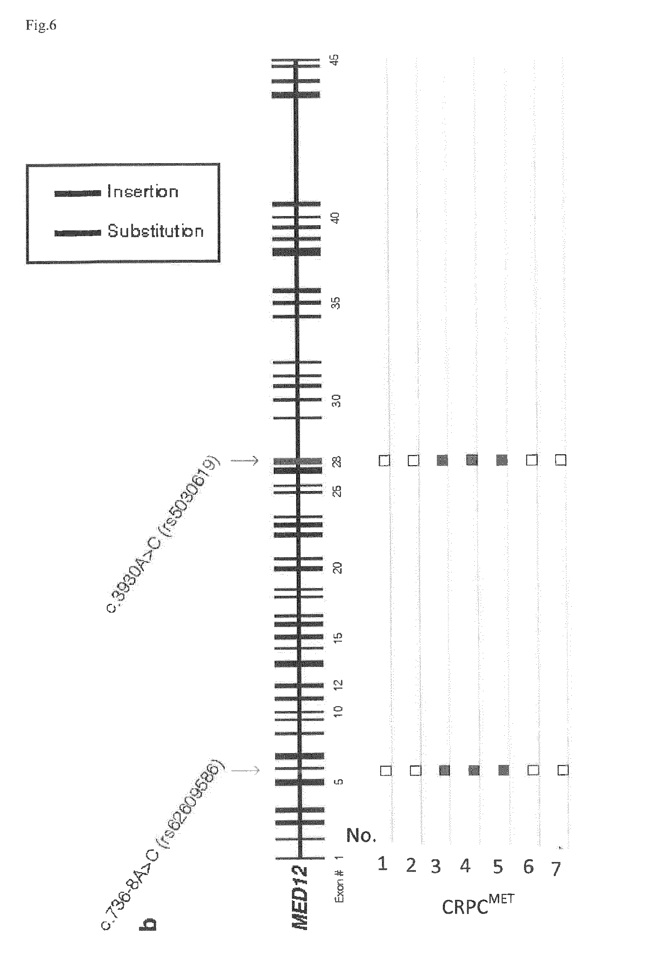

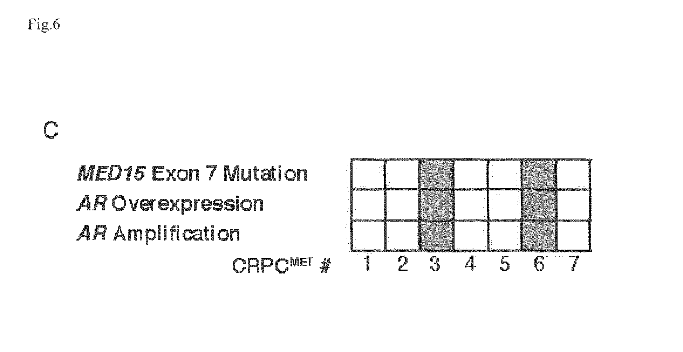

Figure 6: Recurrent somatic alterations in MED15 and MED12 in CRPC^{MET}. a) Detailed view of mutations in MED15 introns and exons detected by Sanger sequencing in a series of 7 CRPC^{MET}. A three base-pair insertion (CAG) was observed in 2 patients in exon 7. Base-pair substitutions were observed in introns 5, 12, 14, and 15 in 1, 4, 3, and 5 patients, respectively. b) Detailed view of mutations in MED12 introns and exons detected by Sanger sequencing in a series of 7 CRPC^{MET}. Base-pair substitutions in both exon 28 and intron 5 were observed in 3 patients. c) Heatmap representation of MED15 exon 7 mutation, AR expression and AR copy number status in a series of 7 CRPC^{MET}.

### Detailed Description

The invention is based on the inventors' surprising finding that mediator 15 (MED15) and/or mediator 12 (MED12) are detectable in prostate cancer tissue whereas both are absent or significantly reduced in healthy and benign control tissue and thus can serve as markers for prostate carcinoma.

The present invention thus relates to methods of diagnosing the risk of developing cancer, determining the prognosis of a patient diagnosed with cancer and identifying a compound for treatment of cancer, using MED15 and/or MED12 as markers for (i) the risk to develop cancer, (ii) an unfavorable prognosis of a patient with cancer, or (iii) identification of compounds for treatment of cancer. In specific embodiments the cancer is prostate cancer. Further, the invention relates to compounds inhibiting MED15 and/or MED12 gene expression or the activity of MED15 and/or MED12 gene products for use in the treatment of cancer.

The MED15 and MED12 markers can be detected in tissue and may also be detected in body fluid samples, e.g., in a blood sample, and thus provide for a novel method for the diagnosis of prostate cancer. As such a method does not require expensive equipment, the costs for diagnosis can thus be reduced. This allows more frequent medical examinations.

As the United States Preventive Services Task Force (USPSTF) recommends against the PSA test for prostate cancer screening in healthy men regardless of age (Moyer VA, on behalf of the U.S. Preventive Services Task, Force (May 2012). "Screening for Prostate Cancer: U.S. Preventive Services Task Force Recommendation Statement." Annals of internal medicine) and the Centers for Disease Control and Prevention shares this conclusion (Prostate Cancer Screening CDC, updated April 6, 2010), there is need in the art for a reliable diagnostic test and treatment of cancer, in particular prostate cancer. The present inventors surprisingly found that MED15 and/or MED12 overexpression is more common in local recurrent castration-resistant prostate cancer (CRPC^{MET}) than androgen receptor (AR) overexpression and ERG rearrangement, and correlate with high proliferative activity. Furthermore, it was found that knock-down of MED15 decreases androgen-dependent and -independent proliferation.

The Mediator of RNA polymerase II transcription subunit 15, also known as Gal11, Spt13 in yeast and PCQAP, ARC105, or TIG-1 in humans is a protein encoded by the MED15 gene. The MED15 gene contains stretches of trinucleotide repeats and is located in the chromosome 22 region which is deleted in DiGeorge's syndrome. Two transcript variants encoding different isoforms have been found for this gene (see http://www.ncbi.nlm.nih.gov/gene: MED15 mediator complex subunit 15 [Homo sapiens (human)]). The human MED15 gene is characterized by Gene ID: 51586 (NCBI). The corresponding proteins are set forth by SEQ ID Nos. 239 and 241 while their mRNAs are set forth by SEQ ID Nos. 240 and 242.

MED15 is a general transcriptional cofactor of the mediator complex involved in RNA polymerase II dependent transcription and found in yeast as an essential factor for Gal4-dependent transactivation (Suzuki Y et al. (1988), Mol. Cell. Biol. 8 (11): 4991-9). Most of the transcription factors that interact with MED15 share the same transactivation domain, 9aaTAD, which directly interacts with the KIX domain of MED15 (Piskacek S. et al. (2007), Genomics 89 (6): 756-68). Human MED15 cooperates in mediator complex (previously known as PC2, ARC, or DRIP) with transcription factors like VP16 and SREBP. It is also known that human MED15 is a subunit of the tail module of the mediator complex and is essential for transforming growth factor-β (TGFβ)/Activin/Nodal/Smad2/3 signal transduction (Kato, Y. et al. (2002), Nature 418, 641-646).

Moreover, the inventors have identified MED12 (human MED12 gene is characterized by Gene ID: 9968 (NCBI) and the corresponding protein is set forth in SEQ ID NO:243; the corresponding mRNA is set forth in SEQ ID NO:244). The MED12 gene, also known as Mediator of RNA polymerase II transcription, subunit 12, is a human gene found on the X chromosome. As MED15, also MED12 is part of the mediator complex and specific interaction of MED12 to Calcitriol receptor, Cyclin-dependent kinase 8, Estrogen receptor alpha, Gli3, G9a, PPARGC1A, MED26, SOX9, and Thyroid hormone receptor alpha are described by different investigators. MED12 is involved in the kinase function of the mediator complex and is a transducer of Wnt/β-catenin signalling which is linked to modulation of hedgehog signalling (Zhou et al. (2006), Mol. Cell. Biol. 26, 8667-8682). Mutations in MED12 are responsible for at least two different forms of X-linked dominant mental retardation, the Lujan-Fryns syndrome and the FG syndrome (Barbieri CE. et al. (2012), Nat. Genet. 44 (6): 685-9).

The inventors have found that the nuclear localization of MED15 and MED12 is specific in human castration-resistant prostate cancer tissue compared to androgen-sensitive controls. For local recurrent castration-resistant prostate cancer (CRPC^{MET}) it is shown that increased expression of MED15 is more common than increased expression of AR which was considered to be the most common event in CRPC^{MET} (Parray, A. et al. (2012), Biologics 6, 267-276). Thus, MED15 and MED12 are useful as diagnostic and prognostic biomarkers in prostate carcinoma and allow improving the management of this disease. Furthermore, the present inventors demonstrated that knock-down of MED15 decreases androgen-dependent and androgen-independent cancer proliferation. Based on these data it can be reasonably expected that MED15 and MED12 can not only serve as diagnostic/prognostic biomarkers but as well as therapeutic targets.

In a first aspect, the present invention relates to a method of diagnosing the risk of developing cancer or determining the prognosis of a patient suffering from cancer, said method comprising:
I.
   (a) determining the level of mediator 15 (MED15) and/or mediator 12 (MED12) in a sample obtained from a patient,
   (b) comparing the data obtained in step a) to a predetermined level of MED15 and/or MED12 observed in a healthy individual, wherein when the level of MED15 and/or MED 12 is increased compared to the predetermined levels in a healthy individual said patient is diagnosed with an increased risk to develop cancer or an unfavorable prognosis, or
II.
   (a) detecting the presence or absence of at least one mutation in the MED15 and/or the MED12 gene in a sample obtained from a patient, wherein said at least one mutation is selected from a group consisting of reference SNP numbers rs117046295, rs361923, rs165643, rs165835, rs6209586 and rs5030619 or a c 1965-10 G>A mutation within the MED15 gene, wherein when at least one of said mutations is detected, said patient is diagnosed with an increased risk to develop cancer or an unfavorable prognosis.

In a second aspect, the present invention relates to the use of MED15 and/or MED12 for diagnosis of cancer or prognosis of a patient suffering from cancer.

In a third aspect, the present invention relates to compounds inhibiting MED15 and/or MED12 gene expression or the activity of MED15 and/or MED12 gene products for use in the treatment of cancer.

In other aspects of the invention, MED15 and/or MED12 are used for diagnosis of cancer or prognosis of a patient suffering from cancer or MED15 and/or MED12 are used to identify a compound for treatment of cancer.

In another aspect, the invention relates to a method of identifying a compound for treatment of cancer, said method comprising (a) contacting cancer cells in culture with a potential cancer modulating compound; (b) determining the level of MED15 and/or MED12 in said cancer cells, and (c) comparing the data obtained in step b) to a predetermined level of MED15 and/or MED12 observed in non-cancer cells or in said cancer cells before the contacting step (a), wherein a decrease in the level of MED15 and/or MED12 indicates that said compound may be useful for the treatment of cancer, thereby determining the activity of said compound to treat cancer.

An increased level of a marker means that its concentration is increased relative to a normal state, i.e. a healthy individual not afflicted by (prostate) cancer. This term includes that in the normal healthy state the marker is not detectable, e.g. is present in levels below the detection limit, but can be detected in cancer patients. It is also possible to define a threshold level, where when the determined level is above this level, it is defined as increased. In a preferred embodiment of the invention the increased marker levels are at least 1.5-times, at least 2-times, at least 3-times, at least 4-times or at least 5-times higher compared to the above described control/normal state.

Determining the prognosis includes risk stratification and prediction of the likelihood of an adverse outcome. This can be made in relation to a certain time period. Adverse outcome in the sense of the present invention include deterioration of a patient's condition, for example due to metastasis, and also death.

Increased likelihood means that compared to an individual where the marker levels are not increased, the chance that a certain event occurs is higher. The increase may be 1 %, 2 %, 3 %, 4 %, 5 %, 7% or 10 %.

MED15 and MED12 are preferably human MED15 and MED12.

In various embodiments, the methods of the invention are limited to determine the level of MED15 or the amount of its gene product.

In various embodiments, the level of MED15 and/or MED12 are determined by determining the amount of mRNA being transcribed from MED15 and/or MED12, preferably the mRNA has the nucleotide sequence set forth in SEQ ID Nos. 240, 242 and/or 244.

In one embodiment, the level of MED15 and/or MED12 are determined by determining the protein amount being expressed from MED15 and/or MED12, preferably the protein has the amino acid sequence set forth in SEQ ID Nos. 239, 241 and/or 243.

Embodiments of the invention also relate to sequencing the MED15 and/or the MED12 gene or to the use of an oligonucleotide probe when the presence or absence of the at least one mutation in the MED15 and/or the MED12 gene is determined.

In various embodiments of the invention the cancer is carcinoma or sarcoma. In more preferred embodiments the cancer is prostate cancer, more preferably a castration-resistant prostate cancer (CRPC). In even more preferred embodiments said cancer is distant metastatic CRPC and/or local recurrent CRPC.

The term "diagnosing cancer", as used herein, relates to the identification of any cancer in an individual. The diagnosis may be based on different identification techniques which comprise, but are not limited to, biopsy, sentinel node biopsy, endoscopy, blood tests, bone marrow aspiration, Pap test, sputum analysis and bronchial washing analysis, imaging studies such as X rays, CT scans, MRI scans and PET scans and genetic analysis.

In some embodiments the patient is a mammal, preferably a human. Generally, the term "mammal", as used herein, comprises human, monkeys, pigs, cows, cats, dogs, guinea pigs, rabbits, mice, sheeps, goats and horses.

The term "cancer", as used herein, relates to a broad group of diseases involving unregulated cell growth. In cancer, cells divide and grow uncontrollably, forming malignant tumours, and invade nearby parts of the body. The cancer may also spread to more distant parts of the body through the lymphatic system or bloodstream. There are over 200 different types of cancer, and each is classified by the type of cell that is initially affected. Cancers are often referred to by terms that contain a prefix related to the cell type in which the cancer originated and a suffix such as -sarcoma (transformed cells of mesenchymal origin), -carcinoma (transformed cells originating in the endodermal or ectodermal germ layer). Common prefixes include prostate-, Adeno- (gland), Chondro- (cartilage), Erythro- (red blood cell), Hemangio- (blood vessels), Hepato- (liver), Lipo- (fat), Lympho- (white blood cell), Melano- (pigment cell), Myelo- (bone marrow), Myo- (muscle), Osteo- (bone), Uro- (bladder), Retino- (eye), and Neuro- (brain).

The term "increased level", as used herein, relates to an increased level of a marker, preferably MED15 and/or MED12. The term describes the level of MED15 and/or MED12 from one or more patients in ratio to levels of the same marker obtained from one or more healthy control individuals. The increased level of a marker may refer to increased amounts of the protein or mRNA. It is also possible to define a threshold level, where when the determined level is above this level, it is defined as increased. In a preferred embodiment of the invention the increased marker levels are at least 1.5-times, at least 2-times, at least 3-times, at least 4-times or at least 5-times higher compared to the above described control/normal state.

The term "increased risk", as used herein, refers to the increased chance a person has, over the course of his or her lifetime, of being diagnosed with or dying from cancer. In a preferred embodiment of the invention the methods of the present invention can determine an increased risk of developing or dying from cancer that is at least 0.5%, at least 1%, at least 2%, at least 3%, at least 5%, at least 10% or at least 20% higher compared to a control individual.

The term "unfavourable prognosis", as used herein, refers to a prediction about a patient's chance of developing or dying from cancer which is higher than the chance of developing or dying from cancer in a control individual. It is also possible to define a threshold level, where when the determined level is above this level, the prognosis is defined as unfavourable. In a preferred embodiment of the invention the prognosis is unfavourable when the patient's level of developing and/or dying from cancer is at least 0.5%, at least 1%, at least 2%, at least 3%, at least 5%, at least 10% or at least 20% higher compared to the one of a control individual. Several numerical prognostic scoring systems are known in the art to determine an individual's prognosis such as the APACHE II scale.

In various embodiments of the invention, the sample is a biological sample, for example a body fluid, cell or tissue sample. Body fluids comprise, but are not limited to blood, blood plasma, blood serum, breast milk, cerebrospinal fluid, cerumen (earwax), endolymph and perilymph, gastric juice, mucus (including nasal drainage and phlegm), peritoneal fluid, pleural fluid, saliva, sebum (skin oil), semen, sweat, tears, vaginal secretion, nipple aspirate fluid, vomit and urine. The cell or tissue sample may comprise material originated from any part of the body such as connective tissue, muscle tissue, nervous tissue, and epithelial tissue. In certain embodiments of the methods detailed above, the tissue sample may be prostate tissue and the cell sample may comprise cells from prostate tissue. The term "obtaining a sample", as used herein, relates to different methods known in the art that comprise, but not limited to, biopsy, sentinel node biopsy or removal of blood, bone marrow, sputum or bronchial fluids.

The term "comparing", as used herein, relates to examination of two or more samples in order to note similarities or differences in the level of MED15 and/or MED12 or to determine mutation in sequence of the MED15 and/or MED12 gene. Typically, one or more samples obtained from patients being tested (e.g. for their cancer status) are "compared" to samples obtained from one or more control individuals. "Control individual" or "healthy individual" as used interchangeably herein, means that any symptoms and/or complications that may relate to a specific disease that is investigated by the methods of the present application such as prostate cancer are absent in said individual. The health status of the control individual may be determined in previous tests known in the art.

"Risk to develop cancer", as used in the present application, refers to the chance a person has, over the course of his or her lifetime (from birth to death), of being diagnosed with or dying from cancer. The term "prognosis" relates to a medical term for predicting the likely outcome of one's current standing, e.g. the survival chance of cancer. Besides the survival rate, a prognosis may also relate to the chance of developing a specific type of cancer, the survival time or the cancer grade of a specific type of cancer. In preferred embodiments of the invention, the methods of the present invention are able to determine a risk to develop and/or to die from cancer that is at least 5%, at least 10%, at least 20%, at least 35%, at least 50%, at least 80%, at least 95% or 100%.

The term "mutation", as used herein, means a change of the nucleotide sequence of the genome of an organism. Mutations result from unrepaired damage to DNA or to RNA genomes (typically caused by radiation or chemical mutagens), from errors in the process of replication, or from the insertion or deletion of segments of DNA by mobile genetic elements. Mutations may or may not produce discernible changes in the observable characteristics (phenotype) of an organism. Moreover, mutations may relate to point mutations, deletions, insertions, duplications or inversion. The term "SNP number", as used herein, relates to the specific single nucleotide polymorphism number of a given mutation referred to in the Single Nucleotide Polymorphism Database (dbSNP) developed and hosted by the National Center for Biotechnology Information (NCBI) in collaboration with the National Human Genome Research Institute (NHGRI).

The term "c 1965-10 G>A mutation within the MED15 gene", as used herein, refers to a point mutation in the MED15 gene. In detail, a guanine base is replaced with an adenine base at the nucleotide position that is located 10 bp before (upstream) exon 16 (in intron 15) of the genomic sequence of the MED15 gene, wherein the first position of exon 16 is nucleotide 1965.

The term "expression", as used herein, relates to a process in which information from a gene is used for the synthesis of a gene product. In cell-based expression systems the expression comprises transcription and translation steps.

The term "nucleic acid molecule" or "nucleic acid sequence", as used herein, relates to DNA (deoxyribonucleic acid) or RNA (ribonucleic acid) molecules. Said molecules may appear independent of their natural genetic context and/or background. The term "nucleic acid molecule/sequence" further refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms.

"At least one", as used herein, relates to one or more, in particular 1, 2, 3, 4, 5, 6, 7, 8, 9, 10 or more.

The term "sequence", as used herein, relates to the primary nucleotide sequence of nucleic acid molecules or the primary amino acid sequence of a protein.

"Culturing", "cultivating" or "cultivation", as used herein, relates to the growth of cells in a specially prepared culture medium under supervised conditions. The term "cancer cell" means cells that grow and divide at an unregulated, quickened pace. In a specific embodiment of the invention the cancer cell may be selected from a group of established cell lines comprising, but are not limited to, 293-T, 721, 9L, A-549, A172, A20, A253, A2780, A2780ADR, A2780cis, A431, B16, B35, BCP-1, BR 293, BxPC3, C6, Cal-27, CHO, CML T1, COS-7, COV-434, CT26, D17, DU145, DuCaP, EM2, EM3, FM3, H1299, HB55, HCA2, HEK-293, HeLa, Hepa1c1c7, HL-60, HMEC, HT-29, HUVEC, J558L, Jurkat, JY, K562, KCL22, KG1, Ku812, KYO1, LNCap, Ma-Mel 1, 2, 3....48, MC-38, MCF-10A, MCF-7, MDA-MB-231, MDA-MB-435, MDA-MB-468, MDCK II, MG63, MONO-MAC 6, MOR/0.2R, MRC5, MTD-1A, MyEnd, NALM-1, NCI-H69/CPR, NCI-H69/LX10, NCI-H69/LX20, NCI-H69/LX4, NW-145, OPCN / OPCT, PC-3, Peer, PNT-1A / PNT 2, Raji, RBL, RenCa, RIN-5F, RMA/RMAS, Saos-2, SiHa, SKBR3, SKOV-3, T-47D, T2, T84, THP1, U373, U87, U937, VCaP, Vero, WM39, WT-49, X63, YAC-1 and YAR cells.

The term "protein", as used herein, relates to one or more associated polypeptides, wherein the polypeptides consist of amino acids coupled by peptide (amide) bonds. The term polypeptide refers to a polymeric compound comprised of covalently linked amino acid residues. The amino acids are preferably the 20 naturally occurring amino acids glycine, alanine, valine, leucine, isoleucine, phenylalanine, cysteine, methionine, proline, serine, threonine, glutamine, asparagine, aspartic acid, glutamic acid, histidine, lysine, arginine, tyrosine and tryptophan.

The term "gene product", as used in the present invention, relates to a biochemical material, either RNA or protein, resulting from expression of a gene. Moreover, the proteins may form complexes with other proteins via covalent and non-covalent bonds.

The term "inhibiting", as used herein, relates to a significant and detectable reduction of protein activity or gene expression activity caused by an effector molecule. Preferred inhibition activities resulting from protein activity or gene expression activity inhibition are more than 10%, 20%, 50%, 80% or 95%.

"RNA" or "ribonucleic acid" as interchangeably used herein relates to a chain of nucleotides wherein the nucleotides contain the sugar ribose and bases selected from the group of adenine (A), cytosine (C), guanine (G), or uracil (U). "DNA" or "deoxyribonucleic acid" as interchangeably used herein relates to a chain of nucleotides wherein the nucleotides contain the sugar 2'-deoxyribose and bases selected from adenine (A), guanine (G), cytosine (C) and thymine (T). The term "mRNA" refers to messenger RNA.

Sequencing of the nucleic acid constructs can be done by the chain termination method, Sanger sequencing or Maxam-Gilbert sequencing or any other technique known in the art. Alternatively, high-throughput sequencing, like pyrosequencing, SOLiD sequencing or DNA nanoball sequencing, is used to determine the sequence of the nucleic acid molecules (Alphey, L. (1997) DNA Sequencing: From Experimental Methods to Bioinformatics, 1st Ed., Bios Scientific Pub Ltd., Oxford, UK).

The present invention employs "probes" or "oligonucleotide probes" that are a fragment of DNA or RNA of variable length (usually 100-1000 bases long) which is used in DNA or RNA samples to detect the presence of nucleotide sequences (the DNA target) that are complementary to the sequence in the probe. Examples of probes which might be used for detection include, but are not limited to, radiolabeled probes, enzymatic labeled probes (e.g. horseradish peroxidase, alkaline phosphatase), affinity labeled probes (e.g. biotin, avidin, or streptavidin) or "molecular beacon" probes. The term "molecular beacon" refers to oligonucleotides such as those sold by Operon Technologies (Alameda, CA) and Synthetic Genetics (San Diego, CA) (see also, Tyagi and Kramer, Nat Biotechnol, 1996, 14:303-308; and Tyagi et al, Nat Biotechnol, 2000, 18:1191-96.). In other embodiments, oligonucleotide probes may be used for partial or complete amplification of oligonucleotides present in the DNA or RNA that is comprised in the sample. Such amplification methods are known in the art and are, for example, described by Faltin et al. (Faltin et al., Clin Chem, 2013, PMID: 23938456).

The term "prostate cancer", as used herein, refers to a form of cancer that originates in the prostate, a gland in the male reproductive system. Most prostate cancers are slow growing; however, there are cases of aggressive prostate cancers. The cancer cells may metastasize (spread) from the prostate to other parts of the body, particularly the bones and lymph nodes. Prostate cancer may cause pain, difficulty in urinating, problems during sexual intercourse, or erectile dysfunction. Other symptoms can potentially develop during later stages of the disease. The term "castration-resistant", as used herein, relates to prostate cancer cells that do not respond to castration, preferably chemical castration. Typically, most hormone dependent cancers become refractory after one to three years and resume growth despite hormone therapy.

The term "contacting" as used herein refers generally to providing access of one component, reagent, analyte or sample to another. For example, contacting can involve mixing a solution comprising a non-nucleic acid receptor with a sample. The solution comprising one component, reagent, analyte or sample may also comprise another component or reagent, such as dimethyl sulfoxide (DMSO) or a detergent, which facilitates mixing, interaction, uptake, or other physical or chemical phenomenon advantageous to the contact between components, reagents, analytes and/or samples.

The term "identify", as used herein, relates to the recognition of a compound that has the ability to completely or partly revert molecular changes of a cell induced by malignant transformation or benign tumour.

The term "compound", as used herein, refers to molecules of different classes such as small molecules according to Lipinski's rule of five, proteins, nucleotides, lipids, sugars and derivatives thereof. In one embodiment, the compound of the present invention can be selected from the group comprising small molecules according to Lipinski's rule of five, proteins and nucleotides. In more preferred embodiments of the invention the compound is an RNAi against MED15 and/or MED12, preferably said RNAi is an siRNA. In other preferred embodiments of the invention the compound is an antibody against MED15 and/or MED12. The term "activity to treat cancer", as used herein, relates to the ability of a compound to completely or partly revert changes induced by cancer. Typically, this may be achieved when the compound inhibits the catalytic activity of an enzyme, blocks the interaction of at least two molecules (e.g. DNA, RNA or proteins) or changes the localization of a protein. Different methods to measure each activity are known in the art.

In certain embodiments, the sample may be subjected to processing before the marker levels are determined. In one embodiment, the sample can, for example, be fractionated to enrich proteins of the nucleus.

For the detection of the markers of the present invention specific binding partners may be employed. In some embodiments, the specific binding partners are useful to detect the presence of a marker in a sample, wherein the marker is a protein or RNA. The marker and its binding partner represent a binding pair of molecules, which interact with each other through any of a variety of molecular forces including, for example, ionic, covalent, hydrophobic, van der Waals, and hydrogen bonding. Preferably, this binding is specific. "Specific binding" means that the members of a binding pair bind preferentially to each other, i.e. usually with a significant higher affinity than to non-specific binding partners. The binding affinity for specific binding partners is thus usually at least 10-fold, preferably at least 100-fold higher than that for non-specific binding partners.

Exemplary binding partners for the markers of the invention are selected from the group consisting of antibodies, antibody fragments and variants, molecules with antibody-like properties, such as lipocalin muteins or Spiegelmers or aptamers. Antibody fragments and variants include Fv fragments, linear single chain antibodies and the like all of which are known to those skilled in the art.

Accordingly, in some embodiments the level of at least one or both markers is determined on mRNA level. In further embodiments the level of at least one or both markers is determined on protein level. In various embodiments, at least one or both markers are determined at mRNA level and at least one or both markers are determined at protein level. Preferably the mRNA has the nucleotide sequence set forth in SEQ ID Nos. 240, 242 and/or 244. In a further embodiment, preferably the protein has the amino acid sequence set forth in SEQ ID Nos. 239, 241 and/or 243.

If a marker is determined on mRNA level, the mRNA may be the mRNA transcript, a 5'-and/or 3'-truncated mRNA or spliced mRNA forms.

If a marker is determined on protein level, the protein may be the full length protein or a fragment thereof. The protein fragment may be a truncated protein, i.e. lack one or more amino acids at the N-terminus or C-terminus or both. This may be due to post-translational processing or due to the action of proteases present in the cell or the sample. The markers determined in the methods of the invention thus also include naturally occurring fragments, preferably immunogenic fragments. Also, the protein may be posttranslationally modified, e.g., phosphorylated, hydroxylated, glycosylated, N-glycosylated, O-glycosylated, ubiquitinylated, sumoylated, acetylated, methylated, prenylated or sulphated.

In certain embodiments of the methods of the invention, in addition to the level of MED15 and/or MED12 the levels of at least 1, 2, 3, 4, 5, 6, 7, 8, 9, 10, 20, 50 or 100 or more markers are determined.

It is understood that in certain embodiments the methods comprise determining the level of MED15 and MED12 which comprises determining the MED15 and MED12 protein level. In alternative variants of these embodiments, the level of one or more further markers is determined. In some of these embodiments, the level of the one or more markers is determined at protein and/or mRNA level. Accordingly, in some embodiments the methods involve the determination of the level of the markers on protein level only.

The methods detailed above, wherein the level of at least one or more markers is determined on protein level comprise in some embodiments the determination of the protein level by an immunoassay, mass spectrometry, chromatography, Western Blot, or gel electrophoresis.

In some embodiments, the immunoassay may be, but are not limited to an Enzyme-linked Immunosorbent Assay (ELISA), Western blot, agglutination test, biotin/avidin type assays, radioimmunoassays, immunoelectrophoresis and immunoprecipitation. The reactions generally include revealing labels such as fluorescent, chemiluminescent, radioactive, enzymatic labels or dye molecules, or other methods for detecting the formation of a complex between the antigen and the antibody or antibodies reacted therewith. These and further immunoassays are well known in the art (David Wild (Ed.): The Immunoassay Handbook. 3rd ed. Elsevier Science Publishing Company, Amsterdam 2005).

The aforementioned assays may involve separation of unbound protein in a liquid phase from a solid phase support to which antigen-antibody complexes are bound. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like.

More particularly, an ELISA method can be used, wherein the wells of a microtiter plate are coated with an antibody against the protein to be tested. A biological sample containing or suspected of containing the marker is then added to the coated wells. After a period of incubation sufficient to allow the formation of antibody-antigen complexes, the plate(s) can be washed to remove unbound moieties and a detectably labelled secondary binding molecule added. The secondary binding molecule is allowed to react with any captured sample marker protein, the plate washed and the presence of the secondary binding molecule detected using methods well known in the art.

In certain embodiments of the above detailed methods, if the determination is via mass spectrometry, the mass spectrometry may be selected from the group comprising MS measurements using EI, CI, ESI, APLI, APPI and APCI.

The marker determination on protein level employing chromatography may be selected from the group comprising liquid chromatography, HPLC, FPLC, Smart chromatography, gel chromatography, size exclusion chromatography, reverse phase chromatography and ionexchange chromatography (Introduction to Modem Liquid Chromatography, Lloyd R. Snyder, Wiley, 2009).

In various embodiments, if the marker is detected via gel electrophoresis, the gel electrophoresis may be selected from the group, but not limited to agarose gel electrophoresis, sodium dodecyl sulfate poly acryl amide gel electrophoresis (SDS-PAGE), 2D-gel electrophoresis, native gel electrophoresis and quantitative preparative native continuous polyacrylamide gel electrophoresis (QPNC-PAGE).

Of course, in certain embodiments of the methods of the present invention at least two determination methods may be coupled to each other in a subsequent manner. In a variant, a gel electrophoresis may be followed by a mass spectroscopic analysis. Alternatively, a gel electrophoresis may be followed by a Western Blot, a chromatography may be followed by a mass spectroscopic analysis, a chromatography may be followed by an immune assay, e.g. an ELISA.

Where in the methods detailed above a marker is determined on mRNA level, the RNA level may be determined by PCR, gel electrophoresis and/or Northern Blot.

In case the marker level is determined on the RNA level, the detection reagent may be a nucleic acid molecule, such as an oligonucleotide. The oligonucleotide may be a nucleic acid probe that may be labelled to allow detection or may be an oligonucleotide primer that allows amplification of the target molecule.

Methods used for the discovery of molecules inhibiting MED15 and/or MED12 may comprise screening of compound libraries or testing of single compounds. After contacting appropriate cells with the compound to be tested successful inhibition of MED15 and/or MED12 may be monitored determining the mRNA or protein levels of said markers by the methods described above or known in the art.

The present invention is further illustrated by the following examples. However, it should be understood, that the invention is not limited to the exemplified embodiments.

### Examples

### Methods

Tissue samples of 718 PCa patients were assessed. Briefly, the cohort comprised 586 primary PCa of different stages and 160 CRPC (90 local recurrent CRPC [28 with corresponding primary PCa] and 70 distant metastatic CRPC).

### Fluorescence in-situ Hybridisation (FISH)

AR, MED12, MED14 and MED15 FISH amplification-FISH assays were performed using RP11-479J1, RP11-308F2, CTD-2375G6, and CTD-2546E19 (Invitrogen, Paisley, UK) as target probes, respectively. Centromeric reference probes were obtained from MetaSystems (Altlussheim, Germany) and Abbott (Wiesbaden, Germany), ERG break-apart FISH was performed as described by Perner et al. (Perner, S. et al. (2006) Cancer Res 66, 8337-8341).

### Immunohistochemistry (IHC)

IHC was conducted using anti-MED12 (HPA003184, Sigma-Aldrich, St. Louis, MO, USA), anti-MED15 (clone 11566-1-AP, Proteintech, Chicago, USA), anti-PHH3 (CMC36911010, Cell Marque, Rocklin, CA, USA), anti-Ki67 (30-9, Ventana Medical Systems, Tucson, AZ, USA), anti-AR (AR441, Dako, Glostrup, Denmark), and anti-ERG (EPR3864, Ventana Medical Systems, Inc. Tucson, AZ, USA). Quantification of IHC was evaluated according to the International Remelle Score.

### Functional Experiments

DU-145, PC-3, and LNCaP cell lines were obtained from ATCC and cultured according to ATCC guidelines. All three cell lines were subjected to siRNA specific against MED12 and MED15 for knockdown. A non-targeting pool was also used as a control. The knockdown efficiency was determined by western blot and IHC using antibodies specific to the protein of interest. The effect of knockdown on cell proliferation was determined using the MTT assay, and in the case of the LNCaP cells following DHT treatment.

### Sanger sequencing

Sanger sequencing was performed on MED12 and MED15 genes on seven castration resistant prostate cancer samples. The used sequencing primers are set forth by SEQ ID Nos. 1-238. An adapter forward primer sequence of GTAAAACGACGGCCAGT, and an adapter reverse primer sequence GGAAACAGCTATGACCATG were added in front of each primer sequence for formalin-fixed paraffin-embedded (FFPE) material.

Statistical analyses

All data are presented as means ± s.e.m.

### Example 1: Inereased MED15 and MED12 expression in prostate cancer tissue

The present inventors found MED15 and MED12 nuclear expression to be significantly higher in CRPC^{MET} and CRPC^{LOC} as compared to androgen-sensitive PCa which includes non-metastasised primary PCa (PCa^{N0}), lymph node metastasised primary PCa with corresponding lymph node metastases (PCa^{N1}), and an independent primary PCa validation cohort (PCa^{VAL}), as well as benign prostatic tissues which specifically lacked MED15 nuclear expression, and exhibited only a cytoplasmic expression (Fig. 1a-b, f-g).

MED15 was overexpressed at a much higher frequency than MED12 in which 76% (53/70) of CRPC^{MET} and 70% (62/89) of CRPC^{LOC} exhibited MED15 overexpression, compared to 40% (28/70), and 21% (19/90) for MED12, respectively (Fig. 1c). To determine whether MED15 overexpression is a specific event that has emerged as response or to modulate a specific signaling pathway that targets MED15 to drive CRPC, or is part of a general overexpression of the entire tail module subunits, the inventors assessed the expression of MED14 which is a subunit of the tail module directly neighboring MED15. Both androgen-sensitive PCa as well as CRPC lacked MED14 nuclear expression (data not shown), suggesting that MED15 overexpression may be a specific event for a protein being part of the mediator tail module. Interestingly, it was observed that all CRPC^{MET} exhibiting MED12 overexpression simultaneously harbor MED15 overexpression, indicating that MED12 overexpression is associated with MED15 overexpression, while MED15 overexpression can occur independently of MED12 overexpression (Fig. 3i). In light of these tissue findings, the present inventors examined the expression of MED15, MED12, and MED14 in metastatic androgen-sensitive (PC3 and DU145) and CRPC^{MET} (LNCaP and VCaP) as well as a benign prostate cells (BPH-1). It was found in support of said tissue findings that MED15 shows a stronger nuclear expression in PCa cells compared to MED12, whereas MED14 lacked nuclear expression (Fig. 2).

Due to the high frequency of MED15 overexpression that was observed in CRPC, the copy number status of MED15, MED12, and MED14 was examined. MED15 was amplified exclusively in CRPC^{MET} tissues in 4% (1/26), as well as in distant metastatic VCaP and CRPC^{MET}DU145 cells (Fig. le, h-k and Fig. 2). On the other hand, MED12 and MED14 were not amplified, but deleted in 4% (3/69) of PCa^{N1} and 3% (1/34) of CRPC^{MET}, respectively (Fig. 1d, 1-o and Fig. 2).

### Example 2: MED15 and MED12 expression following anti-androgen therapy

Due to the high frequency of MED15 overexpression in 70% (62/89) of anti-androgen treated CRPC^{LOC}, and the frequency of 21% (19/90) for MED12 in the latter (Fig. lc), the expression of MED15 and MED12 in hormone-naive untreated locally recurrent PCa (PCa^{LOC/UT}) tissues was assessed to compare the frequency before and after anti-androgen treatment (Fig. 3a-b, e, and g-h). Interestingly, it was observed that the MED15 expression exclusively increases following anti-androgen therapy (Fig. 3a-b). Specifically, 65% (17/26) of anti-androgen treated CRPC tissues showed a significant increase in MED15 expression, in compare to matched corresponding hormone-naive untreated locally recurrent PCa (PCa^{LOC/UT}) tissues, suggesting that increased expression of MED15 may have emerged or selected for during androgen deprivation therapy (Fig. 3c). It was further found that MED15 overexpression correlates with worse clinical outcome in PCa^{LOC/UT}, indicating that PCa^{LOC/UT} overexpressing MED15 represents a highly lethal phenotype (Fig. 3d). Moreover, when two sub-groups of which one exhibits overexpression of both MED15 and MED12 and the other only overexpresses MED15 were compared (Fig. 3i), the inventors found that the group harboring overexpression of both MED15 and MED12 had the worst survival, thus sheds light into the potential implication of signaling pathways that targets both MED15 and MED12 in this highly lethal sub-group (Fig. 4).

Further, the AR expression and copy number status in CRPC^{MET} was examined (Fig. 3i) as AR amplification is reported to emerge during androgen deprivation therapy (Visakorpi, T. et al. (1995) Nature genetics 9, 401-406). In CRPC^{MET} cases, MED15 overexpression in 76%, AR overexpression and amplification in 63% and 36%, respectively, was observed (Fig. 3i). Taking into account that AR overexpression is considered to be the most common event in CRPC (Parray, A. et al. (2012) Biologics 6, 267-276), the findings of the inventors indicate that MED15 overexpression is a more common event in CRPC than AR overexpression. These observations suggest that MED15 is likely to play a role in CRPC^{MET} harboring AR overexpression, but also in a novel subtype of CRPC^{MET} lacking AR overexpression.

The rearrangement of ERG is common in PCa (Tomlins, S. et al. (2005) Science 310, 644-648). Here, it was found that MED15 and MED12 overexpression are even more common events in CRPC^{MET} than the ERG rearrangement (Fig. 3i). Moreover, CRPC^{MET} harboring both MED15 and MED12 overexpression seem to lack ERG rearrangement (Fig. 3 i).

### Example 3: Knockdown of MED15 and MED12 decreases proliferation of prostate cancer cells

The present analysis revealed that overexpression of both MED15 and MED12 significantly correlated with high proliferative activity in PCa (Fig. 5j). To follow-up on these findings, it was found that knock-down of MED15 and MED12 decreased proliferation in both androgen-dependent dihydrotestosteron (DHT) treated LNCaP and -independent CRPC^{MET}DU145 and PC3 cells (Fig.3 a-i).

### Example 4: Recurrent somatic alterations of MED15 and MED12 in CRPC^{MET}

To have a more comprehensive analysis of MED15 and MED12 in CRPC^{MET} and in light of a recent report that MED12 is mutated in 5.4% of primary PCa (Barbieri, C.E. et al. (2012) Nature genetics 44, 685-689), Sanger sequencing of the entire MED15 (18 exons) and MED12 (45 exons) in 7 CRPC^{MET} was performed. MED15 harboured a recurrent somatic mutation (a three base-pair CAG insertion) residing in exon 7 in 2/7 CRPC^{MET} exhibiting MED15 overexpression, as well as several intronic mutations (Fig. 6a). Sequencing of MED12 revealed a recurrent somatic mutation (an A-C base-pair substitution) in exon 28 in 3/7 of CRPC^{MET} as well as an intronic mutation (Fig. 6b).

Notably, the location of the recurrent somatic mutation in exon 7 of MED15 spans a nucleotide position defining the same somatic mutation which was recently reported in two in one patient with ovarian carcinoma (Network, T.C.G.A.R. (2011) Nature 474, 609-615) and one patient with colorectal carcinoma (Network, T.C.G.A.R. (2012) Nature 487, 330-337). Interestingly, in particular exon 7 of MED15 has been reported to be associated with schizophrenia, and susceptibility for this disease (De Luca, A. et al. (2003) Am J Med Genet B Neuropsychiatr Genet 116B, 32-35). Furthermore, MED15 has been implicated in DiGeorge Syndrome /velocardiofacial Syndrome (Napoli, C. et al. (2012) Biochimie 94, 579-587). In regard to MED12 and in addition to the recurrent mutations found in primary PCa, as well as uterine leiomyomas (Makinen, N. et al. (2011) Science 334, 252-255), MED12 is implicated in schizophrenia, Opitze-Kaveggia Syndrome, and Lujan Syndrome. The MED15 mutation found in CRPC^{MET} may influence the activity of MED15 in relation to its essential role in TGFβ/Activin/Nodal/Smad2/3 signalling (Kato, Y. et al. (2002) Nature 418, 641-646), interactions with diverse factors, and the Mediator complex as a whole, whereas the MED12 mutation may effect MED12 as a transducer of Wnt/β-catenin signalling (Kim, S. et al. (2006) The Journal of biological chemistry 281, 14066-14075), hedgehog signalling (Zhou, H. et al. (2006) Mol Cell Biol 26, 8667-8682), and regulation of TGF-β Receptor signalling (Huang, S. et al. (2012) Cell 151, 937-950). Lastly, it was observed that CRPC^{MET} with a MED15 exon 7 mutation exclusively harboured AR amplification and overexpression as compared to tumours lacking the exon 7 mutation (Fig. 6c).

All documents cited herein, are hereby incorporated by reference in their entirety.

The inventions illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention. The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein. Further embodiments of the invention will become apparent from the following claims.

## Claims

1. Method of diagnosing the risk of developing cancer or determining the prognosis of a patient suffering from cancer, said method comprising:
I.
(a) determining the level of mediator 15 (MED15) and/or mediator 12 (MED12) in a sample obtained from a patient,
(b) comparing the data obtained in step a) to a predetermined level of MED15 and/or MED12 observed in a healthy individual, wherein when the level of MED15 and/or MED 12 is increased compared to the predetermined levels in a healthy individual said patient is diagnosed with an increased risk to develop cancer or an unfavorable prognosis, or
II.
(a) detecting the presence or absence of at least one mutation in the MED15 and/or the MED12 gene in a sample obtained from a patient, wherein said at least one mutation is selected from a group consisting of reference SNP numbers rs117046295, rs361923, rs165643, rs165835, rs6209586 and rs5030619 or a c 1965-10 G>A mutation within the MED15 gene, wherein when at least one of said mutations is detected, said patient is diagnosed with an increased risk to develop cancer or an unfavorable prognosis.

2. The method of claim 1 (I), wherein the level of MED15 gene expression or the amount of its gene products is determined.

3. The method of claim 1 (I), wherein the level of MED15 and/or MED12 are determined by determining the amount of mRNA being transcribed from MED15 and/or MED12, preferably the mRNA has the nucleotide sequence set forth in SEQ ID Nos. 240, 242 and/or 244.

4. The method of claim 1 (I), wherein the level of MED15 and/or MED12 are determined by determining the protein amount being expressed from MED15 and/or MED12, preferably the protein has the amino acid sequence set forth in SEQ ID Nos. 239, 241 and/or 243.

5. The method of claim 1 (II), wherein the presence or absence of said at least one mutation in the MED15 and/or the MED12 gene is determined by sequencing the MED15 and/or the MED12 gene or by using an oligonucleotide probe.

6. The method of any one of claims 1 to 5, wherein the cancer is prostate cancer, preferably castration-resistant prostate cancer (CRPC) and more preferably distant metastatic CRPC or local recurrent CRPC.

7. Method of identifying a compound for treatment of cancer, said method comprising:
(a) contacting cancer cells in culture with a potential cancer modulating compound;
(b) determining the level of MED15 and/or MED12 in said cancer cells, and
(c) comparing the data obtained in step b) to a predetermined level of MED15 and/or MED12 observed in non-cancer cells or in said cancer cells before the contacting step (a), wherein a decrease in the level of MED15 and/or MED12 indicates that said compound may be useful for the treatment of cancer.

8. The method of claim 7, wherein the cancer is prostate cancer, preferably castration-resistant prostate cancer (CRPC) and more preferably distant metastatic CRPC or a local recurrent CRPC.

9. The method of claim 7 or 8, wherein the level of the MED15 gene expression or the amount of its gene products is determined.

10. The method of any one of claims 7 to 9, wherein the level of MED15 and/or MED12 are determined by determining the amount of mRNA being transcribed from MED15 and/or MED12.

11. The method of any one of claims 7 to 10, wherein the level of MED15 and/or MED12 are determined by determining the protein amount being expressed from MED15 and/or MED12.

12. Compound inhibiting MED15 and/or MED12 gene expression or the activity of MED15 and/or MED12 gene products for use in the treatment of cancer, wherein the cancer is prostate cancer, preferably castration-resistant prostate cancer (CRPC) and more preferably distant metastatic CRPC or local recurrent CRPC.
